# EUROPEAN PATENT SPECIFICATION

(11) **EP 1 572 173 B1**
(45) Date of publication and mention of the grant of the patent: **28.04.2010**
(21) Application number: 03813233.8
(22) Date of filing: 03.12.2003
(51) Int. Cl.: A61K 31/00, A61K 31/197, A61P 13/00, A61K 31/195

(54) **ALPHA-2-DELTA LIGAND TO TREAT LOWER URINARY TRACT SYMPTOMS**
ALPHA-2-DELTA-LIGAND ZUR BEHANDLUNG VONSYMPTOMEN DER UNTEREN HARNWEGE
LIGAND ALPHA-2-DELTA POUR TRAITER DES SYMPTOMES DES VOIES URINAIRES INFERIEURES

(30) Priority: 13.12.2002 US 433491 P; 05.02.2003 GB 0302657
(43) Date of publication of application: 14.09.2005
(60) Divisional application: 10154836.0
(73) Proprietor: Warner-Lambert Company LLC, New York, NY 10017 (US)
(72) Inventor: TAYLOR, C. P. Jr., 2800 Plymouth Road, Ann Arbor, MI 48105 (US); THORPE, A. J., 2800 Plymouth Road, Ann Arbor, MI 48105 (US); WESTBROOK, S. L., Sandwich, Kent CT13 9NJ (GB); WUSTROW, D. J., 2800 Plymouth Road, Ann Arbor, MI 48105 (US)
(74) Representative: Ruddock, Keith Stephen
(86) International application number: PCT/IB2003/005729
(87) International publication number: WO 2004/054560

(56) References cited:
- EP-A2- 0 533 352
- WO-A1-00/61135
- WO-A1-99/08670
- WO-A1-03/070237

## Description

This invention relates to a new use of alpha-2-delta ligands and their pharmaceutically acceptable derivatives. In particular it relates to a new use of gabapentin and pregabalin.

Lower urinary tract symptoms (LUTS) comprise three groups of symptoms, which are irritative, obstructive and post micturition symptoms. Irritative symptoms comprise urgency, frequency and nocturia, which can be associated with: overactive bladder (OAB) and benign prostatic hyperplasia (BPH).

Over Active Bladder (OAB) is defined as urgency, with or without urge incontinence, usually with frequency and nocturia [Abrams et al., Neurourology and Urodynamics 21:167-178 (2002)]. Prevalence of OAB in men and women is similar, with approximately 16% of the population of the USA suffering from the condition [Stewart et al, Prevalence of Overactive Bladder in the United States: Results from the NOBLE Program; Abstract Presented at the 2nd International Consultation on Incontinence, July 2001, Paris, France].

The terms OAB. Wet and OAB Dry describe OAB patients with or without urinary incontinence respectively. Until recently, the cardinal symptom of OAB was believed to be urinary incontinence. However, with the advent of the new terms this is clearly not meaningful for the large number of sufferers who are not incontinent (i.e. OAB Dry patients). Thus, a recent study from Liberman et al [Health Related Quality of Life Among Adults with Symptoms of Overactive Bladder. Results From A US Community-Based Survey; Urology 57(6), 1044-1050, 2001] examined the impact of all OAB symptoms on the quality of life of a community-based sample of the US population. This study demonstrated that individuals suffering from OAB without any demonstrable loss of urine have an impaired quality of life when compared with controls.

BPH is a chronically progressive disease that can lead to complications such as acute urinary retention, recurrent urinary tract infections, bladder stones and renal dysfunction. The prevalence and average severity of LUTS associated with BPH in men increases with age.

BPH leads to an increase in prostate volume, creating urethral and bladder outflow obstruction as well as secondary changes in bladder function. The effects of this are manifested by both storage (irritative) and voiding (obstructive) symptoms.

Alpha-2-delta ligands have been described for a number of indications. The best known alpha-2-delta ligand, gabapentin (I), known as Neurontin^{®}, 1-(aminomethyl)-cyclohexylacetic acid, was first described in the patent literature in the patent family comprising US4024175.

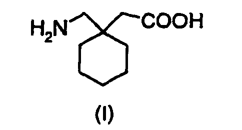

The compound is approved for the treatment of epilepsy and neuropathic pain.

A second alpha-2-delta ligand, pregabalin (II), (S)-(+)-4-amino-3-(2-methylpropyl)butanoic acid, is described in European patent application publication number EP641330 as an anti-conwlsant treatment useful in the treatment of epilepsy and in EP0934061 for the treatment of pain.

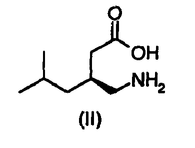

More recently, International Patent Publication Number WO 02/085839, describes alpha-2-delta ligands of the following formulae, for use in the treatment of a number of indications, including pain: 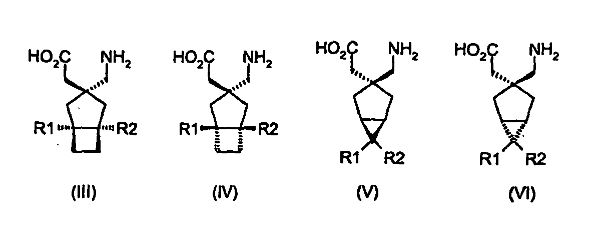 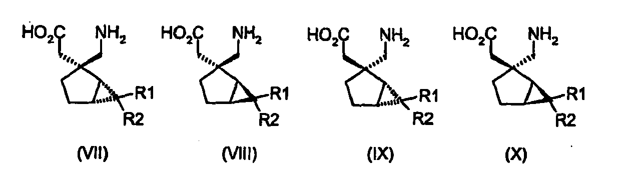 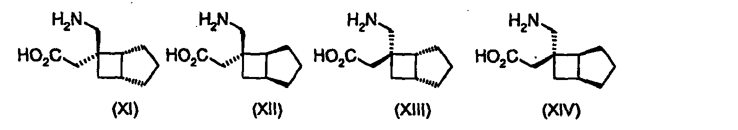 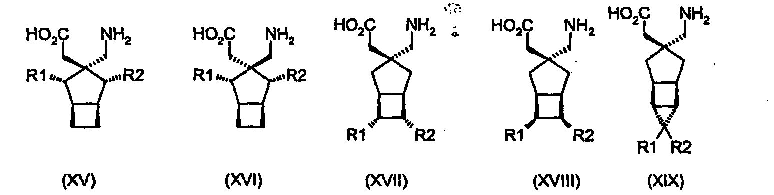 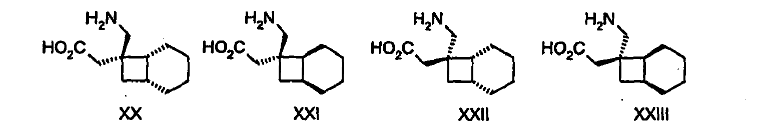 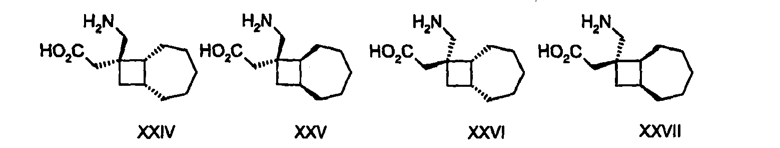 wherein R¹ and R² are each independently selected from H, straight or branched alkyl of 1-6 carbon atoms, cycloalkyl of from 3-6 carbon atoms, phenyl and benzyl, subject to the proviso that, except in the case of a tricyclooctane compound of formula (XVIII), R¹ and R² are not simultaneously hydrogen.

Further examples of alpha-2-delta ligands are the compounds depicted below:

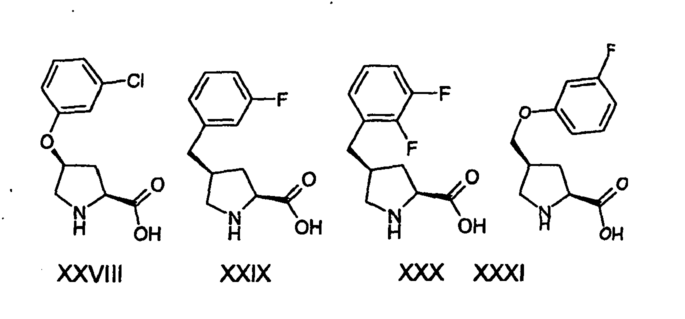

Useful cyclic alpha-2-delta ligands of the present invention are illustrated by the following formula (I): 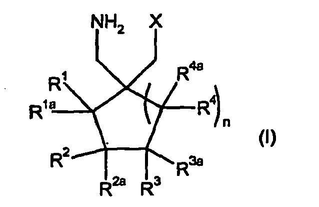 wherein X is a carboxylic acid or carboxylic acid bioisostere;
n is 0, 1 or 2; and
R¹, R^{1a}, R², R^{2a}, R³, R^{3a}, R⁴ and R^{4a} are independently selected from H and C₁-C₆ alkyl, or R¹ and R² or R² and R³ are taken together to form a C₃-C₇ cycloa/kyl ring, which is optionally substituted with one or two substituents selected from C₁-C₆ alkyl, or a pharmaceutically acceptable salt thereof.

In formula (I), suitably, R¹, R^{1a}, R^{2a}, R^{3a}, R⁴ and R^{4a} are H and R² and R³ are independently selected from H and methyl, or R^{1a}, R^{2a}, R^{3a} and R^{4a} are H and R¹ and R² or R² and R³ are taken together to form a C₃-C₇ cycloalkyl ring, which is optionally substituted with one or two methyl substituents. A suitable carboxylic acid bioisostere is selected from tetrazolyl and oxadiazolonyl. X is preferably a carboxylic acid.

In formula (I), preferably, R¹, R^{1a}, R^{2a}, R^{3a}, R⁴ and R^{4a} are H and R² and R³ are independently selected from H and methyl, or R¹⁸, R^{2a}, R^{3a} and R^{4a} are H and R¹ and R² or R² and R³ are taken together to form a C₄-C₅ cycloalkyl ring, or, when n is 0, R¹, R^{1a}, R^{2a}, R^{3a}, R⁴ and R^{4a} are H and R² and R³ form a cyclopentyl ring, or, when n is 1, R¹, R^{1a}, R^{2a}, R^{3a}, R⁴ and R^{4a} are H and R² and R³ are both methyl or R¹, R^{1a}, R^{2a}, R^{3a}, R⁴ and R^{4a} are H and R² and R³ form a cyclobutyl ring, or, when n is 2, R¹, R¹⁸, R², R^{2a}, R³, R^{3a}, R⁴ and R^{4a} are H, or, n is 0, R¹, R^{1a}, R^{2a}, R^{3a}, R⁴ and R^{4a} are H and R² and R³ form a cyclopentyl ring.

Useful acyclic alpha-2-delta ligands of the present invention are illustrated by the following formula (II): 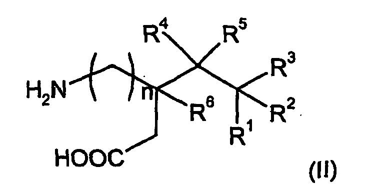 wherein:
n is 0 or 1, R¹ is hydrogen or (C₁-C₆)alkyl; R² is hydrogen or (C₁-C₆)alkyl; R³ is hydrogen or (C₁-C₆)alkyl; R⁴ is hydrogen or (C₁-C₆)alkyl; R⁵ is hydrogen or (C₁-C₆)alkyl and R² is hydrogen or (C₁-C₆)alkyl, or a pharmaceutically acceptable salt thereof.

According to formula (II), suitably R¹ is C₁-C₆ alkyl, R² is methyl, R³ - are hydrogen and n is 0 or 1. More suitably R¹ is methyl, ethyl, n-propyl or n-butyl, R² is methyl, R³ - R⁶ are hydrogen and n is 0 or 1. When R² is methyl, R³ - R⁶ are hydrogen and n is 0, R¹ is suitably ethyl, n-propyl or n-butyl. When R² is methyl, R³ - R⁶ are hydrogen and n is 1, R¹ is suitably methyl or n-propyl. Compounds of formula (II) are suitably in the 33,5R configuration.

Examples of alpha-2-delta ligands for use with the present invention are those compounds generally or specifically disclosed in US4024175, particularly gabapentin, EP641330, particularly pregabalin, US5563175, WO9733858 WO9733859, WO9931057, WO9931074, WO9729101, WO02085839, particularly [(1R,5R,6S)-6-(Aminomethyl)bicyclo[3.2.0]hept-6-yl]acetic acid, WO9931075, particularly 3-(1-Aminomethyl-cyclohexylmethyl)- 4H-[1,2,4]oxadiazol-5-one and C-[1-(1H-Tetrazol-5-ylmethyl)cycloheptyl]-methylamine, WO9921824, particularly (3S,4S)-(1-Aminomethyl-3,4-dimethyl-cyclopentyl)-acetic acid, WO0190052, WO0128978, particularly (1α,3α,5α)(3-amino-methyl-bicyclo[3.2.0]hept-3-yl)-acetic acid, EP0641330, WO9817627, WO0076958, particularly (3S,5R)-3-aminomethyl-5-methyl-octanoic acid, PCT/IB03/00976, particularly (3S,5R)-3-amino-5-methyl-heptanoic acid, (3S,5R)-3-amino-5-methyl-nonanoic acid and (3S,5R)-3-Amino-5-methyl-octanoic acid, EP1178034, EP1201240, WO9931074, WO03000642, WO0222568, WO0230871, WO0230881, WO02100392, WO02100347, WO0242414, WO0232736 and WO0228881 or pharmaceutically acceptable salts thereof

Preferred alpha-2-delta ligands of the present invention include: gabapentin, pregabalin, [(1R,5R,65)-6-(Aminomethyl)bicyclo[3.2.0]hept-6-yl]acetic acid, 3-(1-Aminomethyl-cyclohexylmethyl)-4H-[1,2,4]oxadiazol-5-one, C-[1-(1H-Tetrazol-5-ylmethyl)-cycloheptyl]-methylamine, (3S,4S)-(1-Aminomethyl-3,4-dimethyl-cyclopentyl)-acetic acid, (1α,3α,5α)(3-amino-methyl-bicyclo[3.2.0]hept-3-yl)-acetic acid, (3S,5R)-3-Aminomethyl-5-methyl-octanoic acid, (3S,5R)-3-amino-5-methyl-heptanoic acid, (3S,5R)-3-amino-5-methyl-nonanoic acid and (3S,5R)-3-Amino-5-methyl-octanoic acid, or pharmaceutically acceptable salts thereof. Particularly preferred alpha-2-delta ligands of the present invention are selected from gabapentin, pregabalin and (1α,3α,5α)(3-amino-methyl-bicyclo[3.2.0]hept-3-yl)-acetic acid, or pharmaceutically acceptable salts thereof.

WO 00/01135 describes the use of analogs of glutamic acid and gamma-aminobutyric acid as being useful in treating incontinence.

Surprisingly, it has been found that alpha-2-delta ligands, such as those described above, are useful in the treatment of LUTS, other than urinary incontinence, associated with OAB and/or BPH. More particularly, it has been found that alpha-2-delta ligands are useful in treating the frequency aspect of LUTS associated with OAB and/or BPH. This finding is surprising because it could not have been predicted that a compound known to be useful in the treatment of urinary incontinence (i.e. the unwanted and often unconscious leaking of urine due to problems with muscular control) would be able to reduce the frequency symptoms endured by sufferers of OAB and BPH.

Thus, in accordance with the present invention there is provided the use of an alpha-2-delta ligand, or a pharmaceutically acceptable salt or solvate thereof, for the treatment of LUTS, other than urinary incontinence, associated with OAB and/or BPH.

Preferably the LUTS is frequency. Preferably, the LUTS is associated with BPH. Preferably, when the LUTS is associated with OAB, the OAB is OAB Dry.

Preferably the alpha-2-delta-1-ligand is selected from: 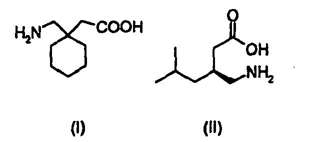 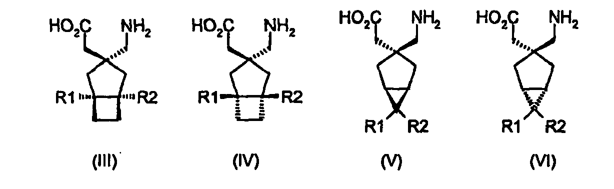 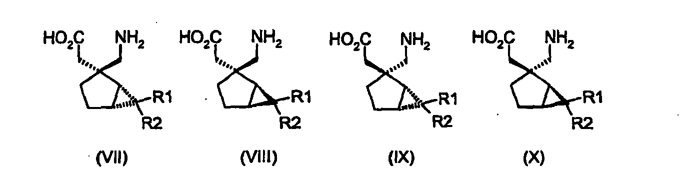 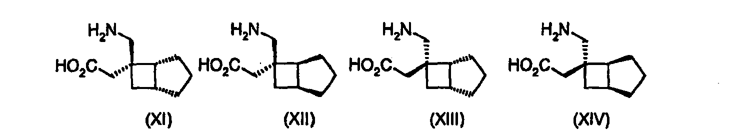 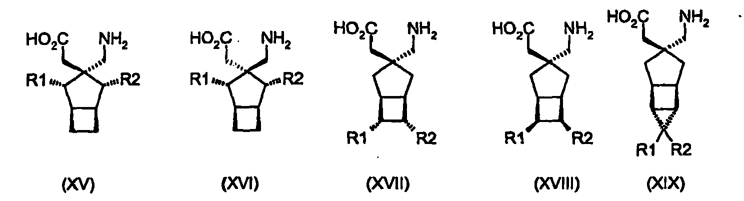 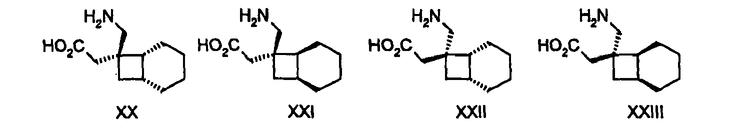 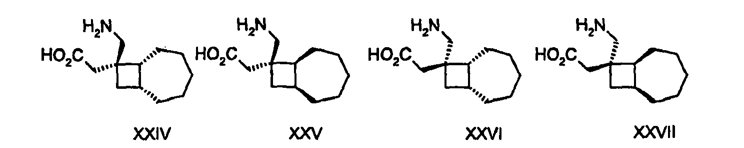 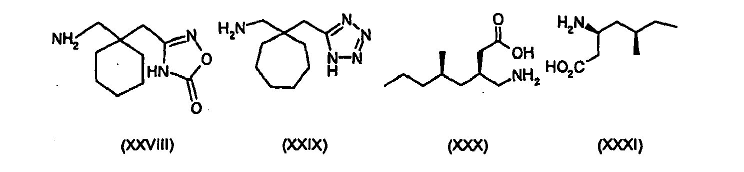 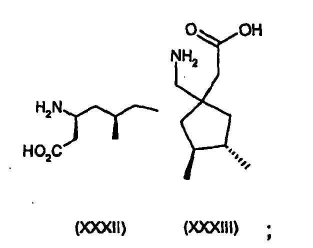 or a pharmaceutically acceptable salt or solvate thereof, wherein R¹ and R² are each independently selected from H, straight or branched alkyl of 1-6 carbon atoms, cycloalkyl of from 3-6 carbon atoms, phenyl and benzyl, subject to the proviso that, except in the case of a tricyclooctane compound of formula (XVIII), R¹ and R² are not simultaneously hydrogen; or it is selected from 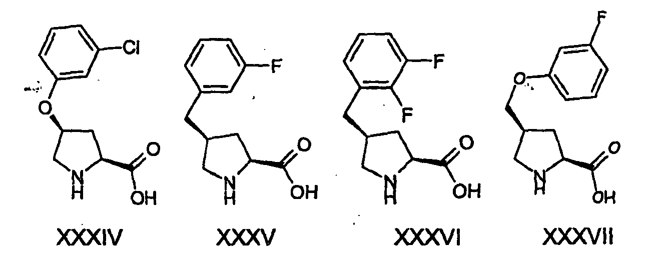 or a pharmaceutically acceptable salt or solvate thereof.

More preferably the alpha-2-delta ligand is gabapentin (I), pregabalin (II) or (1α,3α,5α)(3-amino-methyl-bicyclo[3.2.0]hept-3-yl)acetic acid (III') 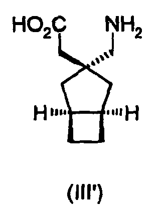 or a pharmaceutically acceptable salt or solvate thereof.

The alpha-2-delta ligand, or pharmaceutically acceptable derivative thereof, can be administered alone or in any convenient pharmaceutical presentation. Oral administration is preferred. In the present indication, a suitable dosage of the alpha-2-delta ligand, or of the active moiety in a pharmaceutically acceptable derivative thereof, is from about 5 to 50 mg/kg of body weight, and preferably about 0.1 to 200 mg/kg.

The invention related to a method of treating LUTS, other than urinary incontinence, associated with OAB and/or BPH comprising administering an alpha-2-delta ligand, or pharmaceutically acceptable salt or solvate thereof, to a patient in need of such treatment.

Alpha-2-delta ligands, particularly the compounds described above, may be used in combination with other compounds. For example, they may be used in combination with α1-Adrenergic antagonists.

The LUTS is other than urinary incontinence. More preferably, the LUTS is frequency. Preferably the LUTS is associated with BPH. Preferably, when the LUTS is associated with OAB, it is OAB dry.

α₁-Adrenergic receptor antagonists useful for combining with alpha-2-delta ligands include, but are not limited to,
(i) terazosin (US Patent No. 4,026,894);
(ii) doxazosin (US Patent No. 4,188,390);
(iii) prazosin (US Patent No. 3,511,836);
(iv) bunazosin (US Patent No. 3,920,636);
(v) alfuzosin (US Patent No. 4,315,007);
(vi) naftopidil (US Patent No. 3,997,666);
(vii) tamsulosin (US Patent No. 4,703,063);
(viii) silodosin (US Patent No. 5,387,603); or
(ix) the compounds disclosed in International Patent Application Publication No. WO 98/30560 (particularly example 19 and its mesylate salt).

Compounds having NRI and/or SRI activity which are useful in combination with alpha-2-delta ligand in the treatment of LUTS associated with OAB and/or BPH include, but are not limited to, the compounds below:
(i) Fluoxetine, fluvoxamine, paroxetine, sertraline, citalopram, vanlafaxine, nefazodone, trazodone, duloxetine and reboxetine (in both racemic and enantiomerically pure forms, e.g. S,S-reboxetine); and
(ii) The compounds disclosed in European Patent Publication No's 1220831 and 1154984, U.S. patent No. 4,018,830, International Patent Publication No. WO 97/17325, U.S. Patent Nos. 5,190,965, 5,430,063, 4,161,529 and U.S. Provisional Application No. 60/121313.

HMG Co-A Reductase inhibitors useful in combination with alpha-2-delta ligand in the treatment of LUTS associated with OAB and/or BPH include, but are not limited to, the compounds below:
(i) Fluvastatin sodium, [R*, S*, -(E)]-(+)-7-[3-(4-fluorophenyl)-1-(1-methylethyl)-H-indol-2-yl]-3,5-dihydroxy-6-heptenoic acid, monosodium salt;
(ii) Cerivastatin sodium, [S-[R*, S*-(E)]-7-[4-(4-fluorophenyl)-5-methoxymethyl)-2,6-bis(1-methylethyl)-3-pyridinyl]-3,5-dihydroxy-6-heptenoate;
(iii) Atorvastatin calcium, [R-(R*, R*)]-2-(4-fluorophenyl)-beta, delta-dihydroxy-5-(1-methylethyl)-3-phenyl-4-[(phenylamino)carbonyl]-1H-pyrrole-1-heptanoic acid, calcium salt (2:1) trihydrate;
(iv) Lovastatin, {S-[1-alpha(R*), 3-alpha, 7-beta, 8-beta (2S*, 4S*), 8a beta]}-1,2,3,7,8,8a-hexahydro-3,7-dimethyl-8-[2-(tetrahydro-4-hydroxy-6-oxo-2H-pyran-2-yl)ethyl-1-naphthalenyl-2-methylbutanoate;
(v) Pravastatin sodium, 1-naphthalene-heptanoic acid, 1,2,6,7,8,8a-hexahydro-beta, delta,6-trihydroxy-2-methyl-8-(2-methyl-1-oxobutoxy)-, monosodium salt, {1S-[1alpha(betas*, deltas*), 2 alpha, 6 alpha, 8 beta(R*), 8a alpha]}; and
(vi) Simvastatin, butanoic acid,2,2-dimethyl-1,2,3,7,8,8a-hexahydro-3,7-dimethyl-8-[2-(tetrahydro-4-hydroxy-6-oxo-2H-pyran-2-yl)-ethyl]-1-naphthalenyl ester, {1S-[1alpha,3alpha,7beta,8beta(2S*,4S*),-8 a beta]}

PDEV inhibitors useful in combination with alpha-2-delta ligand in the treatment of LUTS associated with OAB and/or BPH include, but are not limited to:
(i) The PDE5 inhibitors mentioned in International Patent Application publication nos. WO03/000691; WO02/64590; WO02/28865; WO02/288 WO02/38563; WO02/36593 WO02/28858; WO02/00657 WO02/00656; WO02/1016 WO02/00658; WO01/94347; WO01/94345; WO00/15639 and WO00/15228;
(ii) The PDE5 inhibitors mentioned in US Patents 6,143,746; 6,143,747 and 6,043,252;
(iii) the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the hexahydropyrazino [2',1':6,1]pyrido [3,4-b]indole-1,4-diones disclosed in published international application WO95/19978 the pyrazolo [4,3-d]pyrimidin-4-ones disclosed in WO00/27848; the imidazo[5,1-f][1,2,4]triazinones disclosed in EP-A-1092719 and in published international application WO 99/24433 and the bicyclic compounds disclosed in published international application WO 93/07124; the pyrazolo [4,3-d]pyrimidin-7-ones disclosed in published international application WO 01/27112; the pyrazolo [4,3-d]pyrimidin-7-ones disclosed in published international application WO 01/27113; the compounds disclosed in EP-A-1092718 and the compounds disclosed in EP-A-1092719; the tricyclic compounds disclosed in EP-A-1241170; the alkyl sulphone compounds disclosed in published international application WO 02/074774; the compounds disclosed in published international application WO 02/072586; the compounds disclosed in published international application WO 02/079203 and the compounds disclosed in WO 02/074312:
(iv) Preferably 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil, e.g. as sold as Viagra®) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);5-(2-ethoxy-5-morpholinoacetylphenyl)-1-methyl-3-n-propyl-1,6-dihydro-7H-pyrazolo[4,3-d]pyrimidin-7-one (see EP-A-0526004);3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166); ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one(see WO99/54333); (+)-3-ethyl-5-[5-(4-ethylpiperazin-1-ylsulphonyl)-2-(2-methoxy-1 (R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see WO 01/27113, Example 8);5-[2-iso-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one(see WO 01/27113, Example 15);5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO 01/27113, Example 66);5-(5-Acetyl-2-propoxy-3-pyridinyl)-3-ethyl-2-(1-isopropyl-3-azetidinyl)-2,6-dihydro-7*H*-pyrazolo[4,3-d]pyrimidln-7-one (see WO 01/27112, Example 124); 5-(5-Acetyl-2-butoxy-3-pyridinyl)-3-ethyl-2-(1-ethyl-3-azetidinyl)-2,6-dihydro-7*H*-pyrazolo[4,3-d]pyrimidin-7-one (see WO 01/27112, Example 132); (6R,12aR)-2,3,6,7,12,12a-hexahydro-2-methyl-6-(3,4-methylenedioxyphenyl)pyrazino[2',1':6,1]pyrido[3,4-b]indole-1,4-dione (tadalafil, IC-351, Cialis®), i.e. the compound of examples 78 and 95 of published international application WO95/19978 as well as the compound of examples 1, 3, 7 and 8; 2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil, LEVITRA ®) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e. the compound of examples 20, 19, 337 and 336 of published international application WO99/24433;the compound of example 11 of published international application WO93/07124 (EISAI); compounds 3 and 14 from Rotella D P, J. Med. Chem., 2000, 43, 1257; 4-(4-chlorobenzyl)amino-6,7,8-trimethoxyquinazoline; N-[[3-(4,7-dihydro-1-methyl-7-oxo-3-propyl-1H-pyrazolo[4,3-d]-pyrimidin-5-yl)-4-propxyphenyl]sulfonyl]-1-methyl2-pyrrolidinepropanamide ["DA-8159" (Example 68 of WO00/27848)]; and 7,8-dihydro-8-oxo-6-[2-propoxyphenyl]-1*H-*imidazo[4,5-g]quinazoline and 143-[1-[(4-fluorophenyl)methyl]-7,8-dihydro-8-oxo-1*H*-imidazo[4,5-g]quinazolin-6-yl]-4-propoxyphenyl]carboxamide.
(v) 4-bromo-5-(pyridylmethylamino)-6-[3-(4-chlorophenyl)-propoxy]-3(2H)pyridazinone; 1-[4-[(1,3-benzodioxol-5-ylmethyl)amiono]-6-chloro-2-quinozolinyl]-4-piperidine-carboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a- octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3- (2H)pyridazinone; 1-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)amino]-6-chloro-2- quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer); FR229934 and FR226807 (Fujisawa); and Sch-51866.

Preferably the PDEV inhibitor is selected from sildenafil, tadalafil, vardenafil, DA-8159 and 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one.

Most preferably the PDE5 inhibitor is sildenafil and pharmaceutically acceptable salts thereof. Sildenafil citrate is a preferred salt.

Muscarinic antagonists useful in combination with alpha-2-delta ligand in the treatment of LUTS associated with OAB and/or BPH include, but are not limited to:
(i) Atropine, fluvoxate, hyoscine, oxybutynin, tolterodine and the compounds disclosed in International Patent Publication No. WO 89/06644, propantheline, propiverine, trospium, and the compounds disclosed in International Patent Publication No. WO 98/05641, along with the pharmaceutically acceptable salts thereof..
(ii) Especially preferred are darifenacin, oxybutynin, tolterodine and the compounds disclosed in International Patent Publication No. WO 89/06644, and the compounds disclosed in International Patent Publication No. WO 98/05641, along with the pharmaceutically acceptable salts thereof.

The muscarinic antagonist can be selective for M₃ receptors or it can be non-selective, exhibibng antagonism at M₁, M₂ and M₃. Antagonists selective for the M₃ receptor are preferred.

In particular tolterodine is of interest.

COX inhibitors useful in combination with alpha-2-delta ligand in the treatment of LUTS associated with OAB and/or BPH include, but are not limited to:
(i) ibuprofen, naproxen, benoxaprofen, flurbiprofen, fenoprofen, fenbufen, ketoprofen, indoprofen, pirprofen, carprofen, oxaprozin, prapoprofen, miroprofen, tioxaprofen, suprofen, alminoprofen, tiaprofenic acid, fluprofen, bucloxic acid, indomethacin, sulindac, tolmetin, zomepirac, diclofenac, fenclofenec, alclofenac, ibufenac, isoxepac, furofenac, tiopinac, zidometacin, acetyl salicylic acid, indometacin, piroxicam, tenoxicam, nabumetone, ketorolac, azapropazone, mefenamic acid, tolfenamic acid, diflunisal, podophyllotoxin derivatives, acemetacin, droxicam, floctafenine, oxyphenbutazone, phenylbutazone, proglumetacin, acemetacin, fentiazac, clidanac, oxipinac, mefenamic acid, meclofenamic acid, flufenamic acid, niflumic acid, flufenisal, sudoxicam, etodolac, piprofen, salicylic acid, choline magnesium trisalicylate, salicylate, benorylate, fentiazac, clopinac, feprazone, isoxicam and 2-fluoro-a-methyl[1,1'-biphenyl]-4-acetic acid, 4-(nitrooxy)butyl ester (See Wenk, et al., Europ. J. Pharmacol. 453:319-324 (2002));
(ii) meloxicam, (CAS registry number 71125-38-7; described in U.S. Patent No. 4,233,299), or a pharmaceutically acceptable salt or prodrug thereof;
(iii) Substituted benzopyran derivatives that are described in U.S. Patent No. 6,271,253. Also benzopyran derivatives described in U.S. Patent Nos. 6,034,256 and 6,077,850 along with international Publication No's WO 98/47890 and WO 00/23433;
(iv) Chromene COX2 selective inhibitors described in U.S. Patent No. 6,077,850 and U.S. Patent No. 6,034,256;
(v) The compounds described in International Patent Application Publication No's WO 95/30656, WO 95/30652, WO 96/38418 and WO 96/38442, and the compounds described in European Patent Application Publication No. 799823, along with the pharmaceutically acceptable derivatives thereof;
(vi) celecoxib (US Patent No. 5,466,823), valdecoxib (US Patent No. 5,633,272), deracoxib (US Patent No. 5,521,207), rofecoxib (US Patent No. 5,474,995), etoricoxib (international Patent Application Publication No. WO 98/03484), JTE-522 (Japanese Patent Application Publication No. 9052882), or a pharmaceutically acceptable salt or prodrug thereof;
(vii) Parecoxib (described in U.S. Patent No. 5,932,598), which is a therapeutically effective prodrug of the tricyclic Cox-2 selective inhibitor valdecoxib (described in U.S. Patent No. 5,633,272), in particular sodium parecoxib;
(viii) ABT-963 (described in International Patent Application Publication No. WO 00/24719)
(ix) Nimesulide (described in U.S. Patent No. 3,840,597), flosulide (discussed in J. Carter, Exp.Opin.Ther.Patents, 8(1), 21-29 (1997)), NS-398 (disclosed in U.S. Patent No. 4,885,367), SD 8381 (described in U.S. Patent No. 6,034,256), BMS-347070 (described in U.S. Patent No. 6,180,651), S-2474 (described in European Patent Publication No. 595546) and MK-966 (described in U.S. Patent No. 5,968,974);
(x) The compounds and pharmaceutically acceptable derivatives described in U.S. Patent No. 6,395,724, U.S. Patent No. 6,077,868, U.S. Patent No. 5,994,381, U.S. Patent No. 6,362,209, U.S. Patent No. 6,080,876, U.S. Patent No 6,133,292, U.S. Patent No. 6, 369,275, U.S. Patent No. 6,127,545, U.S. Patent No. 6,130,334, U.S. Patent No. 6,204,387, U.S. Patent No. 6,071,936, U.S. Patent No. 6,001,843, U.S. Patent No. 6,040,450, International Patent Application Publication No WO 96/03392, International Patent Application Publication No WO 96/24585, U.S. Patent No. 6,340,694, U.S. Patent No. 6,376,519, U.S. Patent No. 6,153,787, U.S. Patent No. 6,046,217, U.S. Patent No. 6,329,421, U.S. Patent No. 6,239,137, U.S. Patent No. 6,136,831, U.S. Patent No. 6,297,282, U.S. Patent No. 6,239,173, U.S. Patent No. 6,303,628, U.S. Patent No. 6,310,079, U.S. Patent No. 6,300,363, U.S. Patent No. 6,077,869, U.S. Patent No. 6,140,515, U.S. Patent No. 5,994,379, U.S. Patent No. 6,028,202, U.S. Patent No. 6,040,320, U.S. Patent No. 6,083,969, U.S. Patent No 6,306,890, U.S. Patent No. 6,307,047, U.S. Patent No. 6,004,948, U.S. Patent No. 6,169,188, U.S. Patent No. 6,020,343, U.S. Patent No. 5,981,576, U.S. Patent No. 6,222,048, U.S. Patent No. 6,057,319, U.S. Patent No. 6,046,236, U.S. Patent No. 6,002,014, U.S. Patent No. 5,945,539, U.S. Patent No. 6,359,182, International Patent Application Publication No. WO 97/13755, International Patent Application Publication No. WO 96/25928, International Patent Application Publication No. WO 96/374679, International Patent Application Publication No. WO 95/15316, International Patent Application Publication No. WO 95/15315, International Patent Application Publication No. WO 96/03385, International Patent Application No. WO 95/00501, International Patent Application No. WO 94/15932, International Patent Application Publication No. WO 95/00501, International Patent Application Publication No. WO 94/27980, International Patent Application Publication No. WO 96/25405, International Patent Application Publication No. WO 96/03388, International Patent Application Publication No. WO 96/03387, U.S. Patent No. 5,344,991, International Patent Application Publication No. WO 95/00501, International Patent Application Publication No. WO 96/16934, International Patent Application Publication No. WO 96/03392, International Patent Application Publication No. WO 96/09304, International Patent Application Publication No. WO 98/47890, and International Patent Application Publication No. WO 00/24719.

When treating BPH, alpha-2-delta ligands may be combined with a compound that attenuates the growth of the prostate gland. For example, a formulation is envisaged that combines an alpha-2-delta ligand with a human 5-α reductase inhibitory compound [see International Patent Application WO 95/28397].

The use of the compounds described herein may have the advantage that higher potency, longer duration of action, fewer side effects, improved selectivity, or other more useful properties are achieved compared to the uses of the prior art when treating LUTS associated with OAB and/or BPH, particularly when the LUTS is frequency.

The compounds of the present invention are prepared by methods well known to those skilled in the art. Specifically, the patents, patent applications and publications, mentioned hereinabove, exemplify compounds which can be used in combinations, pharmaceutical compositions, methods and kits in accordance with the present inventions, and refer to methods of preparing those compounds.

Pharmaceutically acceptable salts of the compounds suitable for use in the invention include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, Weinheim, Germany, 2002).

A pharmaceutically acceptable salt of a compound suitable for use in the present invention may be readily prepared by mixing together solutions of the compound and the desired acid or base, as appropriate. The salt may precipitate from solution and be collected by filtration or may be recovered by evaporation of the solvent. The degree of ionisation in the salt may vary from completely ionised to almost non-ionised.

The compounds suitable for use in the present invention may exist in both unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds suitable for use in the present invention include references to salts, solvates and complexes thereof and to solvates and complexes of salts thereof.

The compounds suitable for use in the present invention include the compounds as hereinbefore defined, polymorphs, and isomers thereof (including optical, geometric and tautomeric isomers) as hereinafter defined and isotopically-labelled compounds.

As stated, the invention includes all polymorphs of the compounds suitable for use in the present invention as hereinbefore defined.

Compounds suitable for use in the present invention containing one or more asymmetric carbon atoms can exist as two or more stereoisomers. Where a compound contains an alkenyl or alkenylene group, geometric *cisltrans* (or Z/E) isomers are possible. Where the compound contains, for example, a keto or oxime group or an aromatic moiety, tautomeric isomerism ('tautomerism') can occur. It follows that a single compound may exhibit more than one type of isomerism.

Included within the scope of the present invention are all stereoisomers, geometric isomers and tautomeric forms of the compounds suitable for use in the present invention as hereinbefore described, including compounds exhibiting more than one type of isomerism, and mixtures of one or more thereof. Also included are acid addition or base salts wherein the counter-ion is optically active, for example, D-lactate or L-lysine, or racemic, for example, DL-tartrate or DL-arginine.

The present invention includes all pharmaceutically acceptable isotopically-labelled compounds suitable for use in the present invention as hereinbefore described wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ^{3B}Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Pharmaceutically acceptable solvates in accordance with the invention include those wherein the solvent of crystallization may be isotopically substituted, e.g. D₂O, d₆-acetone, d₆-DMSO.

Compounds of the invention intended for pharmaceutical use may be administered as crystalline or amorphous products. They may be administered alone or in combination with one or more other compounds of the invention. Generally, they will be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 wt% to 5 wt% of the tablet, and glidants may comprise from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 wt% to 10 wt%, preferably from 0.5 wt% to 3 wt% of the tablet.

Other possible ingredients include anti-oxidants, colourants, flavouring agents, preservatives and taste-masking agents.

Exemplary tablets contain up to about 80% drug, from about 10 wt% to about 90 wt% binder, from about 0 wt% to about 85 wt% diluent, from about 2 wt% to about 10 wt% disintegrant, and from about 0.25 wt% to about 10 wt% lubricant

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Verma et al, Pharmaceutical Technology On-line, 25(2), 1-14 (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of the present invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

The compounds of the Invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (*e.g*. Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane.

For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or HPMC), blisters and cartridges for use in an inhaler or insufflator may be formulated to contain a powder mix of the compound of the invention, a suitable powder base such as lactose or starch and a performance modifier such as /-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 µg to 20mg of the compound of the invention per actuation and the actuation volume may vary from 1 µl to 100 µl. A typical formulation may comprise a compound of the invention, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly(DL-lactic-coglycolic acid (PGLA).

Modified release formulations include delayed-, sustained-, pulsed-, controlled-, targeted and programmed release.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (*e.g*. absorbable gel sponges, collagen) and non-biodegradable (*e.g*. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in international Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for co-administration of the compositions.

Thus the kit of the invention comprises two or more separate pharmaceutical compositions, at least one of which contains a compound as hereinbefore described in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For the avoidance of doubt, references herein to "treatment" include references to curative, palliative and prophylactic treatment.

### CHEMISTRY EXAMPLES:

### EXAMPLE 1:

### (2S, 4S)-4-(3-Chloro-phenoxy)-pyrrolidine-1,2-dicarboxylic acid 1-tert-butyl ester 2-methyl ester

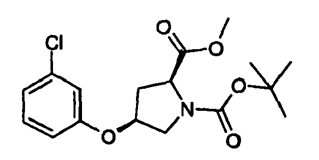

To a stirred solution of (2S, 4R)-4-hydroxy-pyrrolidine-1,2-dicarboxylic acid 1-tert-butyl ester 2-methyl ester (CAS Reg 74844-91-0) (6.1kg,24.87mol), 3-chlorophenol (3.52kg,27.39mol) & triphenylphosphine (7.18kg,27.37mol) in tert-butyl methyl ether (30.5L) at 0°C was added diisopropylazodicarboxylate (5.53kg,27.35mol) in tert-butyl methyl ether (15L) dropwise. The mixture was stirred overnight at 20°C. The reaction was filtered and the liquors washed with 0.5M sodium hydroxide (aq) (2 x 12.5L) & water (12.2L). The tert-butyl methyl ether solvent was replaced with n-heptane (42.7L) by atmospheric pressure distillation & cooled to crystallise crude product, which was collected by filtration (11.1 kg, 125% contaminated with ca 35% reduced diisopropyl dicarboxylate & triphenylphosphine oxide - corrected yield = 86%).
¹H NMR (400MHz, CDCl₃): δ = 1.46, 1.49 (2 x s, 9H), 2.47 (2H, m), 3.71 (5H, m), 4.42 (1H, m), 4.42, 4.54 (1H, 2 x m), 4.87 (1H, m), 6.68 (1H, m), 6.79 (1H, s), 6.92 (1H, m), 7.18 (1H, m).
LRMS (Electrospray): m/z 378 (MNa⁺).

### (2S, 4S)-4-(3-Chloro-phenoxy)-pyrrolidine-1,2-dicarboxylic acid 1-tert-butyl ester

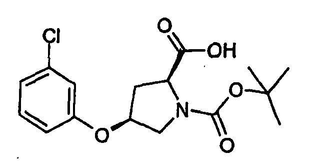

To (2S, 4S)-4-(3-Chloro-phenoxy)-pyrrolidine-1,2-dicarboxylic acid 1-tert-butyl ester 2-methyl ester (11.1kg, 20.28mol) in THF (26.6L) was added a solution of LiOH.H₂O (4.86kg, 115.4mol) in water (55.5L). The mixture was stirred overnight at 25°C. The THF was removed by distillation & the resultant aqueous solution extracted with dichloromethane (33.3L & 16.7L). The combined dichloromethane layers were extracted with water (33L & 16.7L). The combined aqueous phases were adjusted to pH 3-3.5 with 1 M hydrochloric acid(aq) & extracted with dichloromethane (2 x 22.2L). The combined dichloromethane phases were replaced with toluene (33.3L), which was cooled to crystallise the product, which was collected by filtration (6.1 kg, 98%).
¹H NMR (400 MHz, CDCl₃): δ = 1.42, 1.48 (2 x s, 9H), 2.30-2.70 (m, 2H), 3.60-3.80 (m, 2H), 4.40-4.60 (m, 1H), 4.86 (m, 1H), 6.71 (m, 1H), 6.82 (m, 1H), 6.94 (m, 1H), 7.16 (m, 1H).
LRMS (Electrospray): m/z [MNa⁺] 364, 340 [M-1] 340.

### (2S, 4S)-4-(3-Chloro-phonoxy)-pyrroildine-2-carboxylic acid (XXVIII)

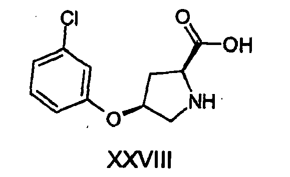

A solution of (2S, 4S)-4-(3-Chloro-phenoxy)-pyrrolidine-1,2-dicarboxylic acid 1-tert-butyl ester (29.25mol) was dissolved in THF (20L) & filtered. To this solution was added 4M HCl in dioxane (30L) & stirred overnight. Tert-Butyl methyl ether (70L) was added to the resultant suspension & the product was collected by filtration (7.06kg, 86.7%).
¹H NMR (400 MHz, CD₃OD): δ = 2.65 (m, 2H), 3.60 (dd, 1H), 3.70 (d, 1H), 4.60 (dd, 1H), 5.02 (m, 1H), 6.88 (m, 1H), 6.97 (s, 1H), 7.03 (d, 1H), 7.29 (dd, 1H). LRMS (Electrospray [MH⁺] 242, [M-1] 240.
Microanalysis: Found, C, 46.97; H, 4.70; N, 4.90. C₁₁H₁₂ClNO₃.HCl.0.1H₂O requires C, 47.20; H, 4.75; N, 5.00.

### EXAMPLE 2:

### 4-(3-Fluoro-benzyl)-pyrrolidine-1,2-dicarboxylic acid 1-tert-butyl ester 2-(2-isopropyl-5-methyl-cyclohexyl) ester

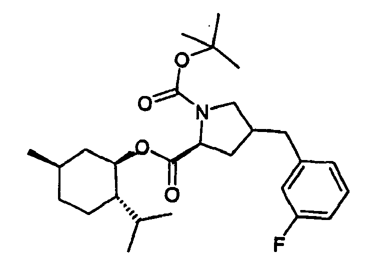

4-(3-Fluoro-benzylidene)-pyrrolidine-1,2-dicarboxylic acid 1-tert-butyl ester 2-(2-isopropyl-5-methyl-cyclohexyl) ester (1.20 g, 2.61 mmol) was dissolved in ethyl acetate:toluene (1:1, 12 ml). The solution was submitted to hydrogenation on platinum oxide (120 mg, 10 % by weight) at 25 °C and 15 psi for 1 hour. The reaction mixture was filtered through arbocel and the filtrate reduced under pressure. The residue was purified by flashmaster chromatography eluting with heptane:ethyl acetate (15:1) to yield the title compound as a colourless oil (1.11 g, 91 %).
¹H-NMR (400MHz, CD₃OD): δ = 0.72-1.37 (m, 13 H), 1.44 (d, 9H), 1.43-1.75 (m, 4H), 1.87-2.01 (m, 2H), 2.31-2.58 (m, 2H), 2.83 (d, 2H), 3.07 (t, 1H), 3.50-3.65 (m, 1H), 4.13-4.30 (dt, 1H), 4.71 (td, 1H), 6.90 (d, 2H), 7.00 (d, 1H), 7.30 (q, 1H).
LRMS (APCI): m/z [MH-BOC]⁺ 362.

### (2S,4S)-4-(3-Fluoro-benzyl)-pyrrolidine-2-carboxylic acid mono hydrochloride salt (XXIX)

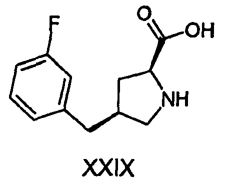

4-(3-Fluoro-benzyl)-pyrrolidine-1,2-dicarboxylic acid 1-tert-butyl ester 2-(2-isopropyl-5-methyl-cyclohexyl) ester (0.91 g, 1.96 mmol) was dissolved in toluene (2 ml). 6N hydrochloric acid (50ml) was added and stirred at reflux for 18 h. The reaction mixture was cooled to room temperature and extracted with ethyl acetate (3 x 20 ml). The aqueous layer was concentrated by evaporated under reduced pressure to give the title compound (417mg, 81 %) as a white solid. ¹H-NMR showed a 7:1 ratio of *cis:trans* diastereoisomers so the product was recrystallised from isopropyl alcohol to give the title compound (170mg, 65%) in a ratio of 14:1 *cis:trans* as determined by NMR.
¹H-NMR (400MHz, CD₃OD): (mixture of diastereoisomers 2*S*,4*S*:2*S*,4*R* (*14:1*)): δ = 1.85 (q, 1H), 2.51 (quin, 1H), 2.69-2.85 (m, 3H), 3.07 (t, 1H), 3.41 (dd, 1H), 4.38 and 4.48 (t, 1H), 6.90-7.04 (m, 3H), 7.32 (q, 1H).
LRMS (APCI): m/z [MH]⁺ 224.
[α]_{D}²⁵ -1.27° (c=9.00 in methanol).
Microanalysis: Found C, 55.56; H, 5.81; N, 5.34%. C₁₂H₁₄FNO₂.HCl requires C, 55.50; H, 5.82; N, 5.39%.

### EXAMPLE 3:

### 4-(3-Fluoro-benzyl)-pyrrolidine-1,2-dicarboxylic acid 1-tert-butyl ester 2-(2-isopropyl-5-methyl-cyclohexyl) ester

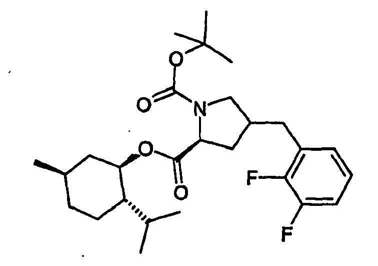

4-(3-Fluoro-benzyl)-pyrrolidlne-1,2-dicarboxylic acid 1-tert-butyl ester 2-(2-isopropyl-5-methyl-cyclohexyl) ester was prepared by a method analogous to that for the preparation of 4-(3-Fluoro-benzyl)-pyrrolidine-1,2-dicarboxylic acid 1-tert-butyl ester 2-(2-isopropyl-5-methyl-cyclohexyl) ester, using the appropriate starting alkenic menthol ester; [MH]480
Microanalysis (mixture of diastereoisomers *cis* (major) and *trans*)*:* Found: C, 67.74; H, 8.30; N, 2.90%. C₂₇H₃₉ F₂NO₄. requires C, 67.62; H, 8.20; N, 2.92%;
[α]_{D}²⁵ -71.92° (c = 3.26 in methanol)

### (2S,4S)-4-(2,3-Difluoro-benzyl)-pyrrolidine-2-carboxylic acid monohydrochloride salt (XXX)

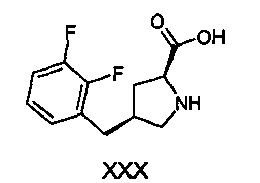

The title compound was made by the method of example 2, starting from 4-(3-Fluorobenzyl)-pyrrolidine-1,2-dicarboxylic acid 1-tert-butyl ester 2-(2-isopropyl-5-methylcyclohexyl) ester, and purified by re-crystallisation with acetone/ether to give the title compound as a mixture of diastereoisomers (2S,4S:2S,4R (12:1)) determined by ¹H-NMR (500 mg, 60 %) as a white solid.
¹H-NMR (400 MHz, CD₃OD) (mixture of diastereoisomers cis:*trans (12:1)*): δ= 0.80-1.90 (m, 0.92H), 2.12-2.20 (m, 0.08H), 2.28-2.36 (m, 0.08H), 2.49-2.58 (q, 0.92H), 2.66-2.81 (m, 1H), 2.83-2.95 (m, 2H), 3.02-3.13 (t, 1H), 3.46 (dd, 1H), 4.40 (dd, 0.92H), 4.48-4.54 (m, 0.08H), 7.03-7.20 (m, 3H).
LRMS (Electrospray): m/z [M + H]⁺ 242.
Microanalysis: Found C, 51.42; H, 5.08; N, 5.01%. C₁₂H₁₃NO₂F₂.HCl requires C, 51.90; H, 5.08; N, 5.04%.

### EXAMPLE 4:

### (2S,4S)-Pyrrolidine-1,2,4-tricarboxylic acid 1,2-di-tert-butyl ester

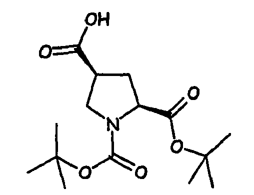

To a mixture of 4-phenyl-pyrrolidine-1,2-dicarboxylic acid di-tert-butyl ester (CAS Reg. No. 344 286-69-7; J. Org. Chem., 2001, 3593-3596) (0.78g, 2.24mmol) and sodium periodate (5.77g, 27mmol) stirring at 0°C under a nitrogen atmosphere in ethyl acetate (5.5ml), acetonitrile (5.5ml) and water (8.5ml) was added ruthenium trichloride (10mg, 0.05mmol) and stirred to room temperature over 18 hours. Diethyl ether (20ml) was added and stirred for a further 1 hr. 1 M hydrochloric acid (5ml) was added and the mixture extracted with ethyl acetate (3 x 30ml). Organic extracts were combined, dried (MgSO₄), filtered and evaporated under reduced pressure. The residue was purified by chromatography on silica gel, eluting with 50:50:1 ethyl acetate:heptane:glacial acetic acid to give the title compound as a colourless gum (501 mg, 78%)
¹H-NMR (400MHz, CDCl₃): δ = 1.40-1.49 (m, 18H); 2.26-2.40 (m, 1H); 2.42-2.56 (m, 1H); 3.02-3.12 (m, 1H); 3.65-3.80 (m, 1.4H) & 3.80-3.88 (m, 0.6H) [rotamers]; 4.09-4.20 (m, 0.7H) & 4.20-4.26 (m, 0.3H) [rotamers]
LRMS (electrospray): [M-1] 314

### (2S,4S)-4-(3-fluoro-phenoxymethyl)-pyrroildine-2-carboxylic acid (XXI)

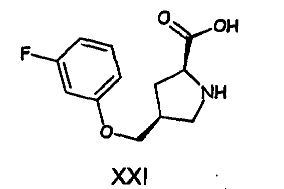

4-(3-fluoro-phenoxymethyl)-pyrroline-1,2-dicarboxylic acid di-tert-butyl ester (475mg, 1.2mmol) was dissolved in a solution of anhydrous hydrogen chloride in dioxane (4M, 15ml) and stirred at 50°C under a nitrogen atmosphere for 1 hour. The solvent was removed under reduced pressure and the resulting semi-solid triturated with ethyl acetate to give a white solid which was recrystallised from ethyl acetate/isopropyl alcohol to give the title compound as a mixture of diastereomers (-5:1 2S,4S:2S,4R) as a white solid hydrochloride salt (90mg, 35%)
¹H-NMR (400MHz, CD₃OD): δ = 2.04-2.09 (m, 0.8H); 2.33-2.47 (m, 0.4H); 2.65-2.75 (m, 0.8H); 2.88-3.00 (m, 1H); 3.33-3.40 (m, 1H); 3.52-3.60 (m, 0.8H); 3.60-3.68 (0.2H); 3.96-4.04 (m, 1H); 4.04-4.12 (m, 1H); 4.42-4.51 (m, 0.8H); 4.40-4.56 (m, 0.2H); 6.65-6.80 (m, 3H), 7.21-7.30 (m, 1H)
LRMS (electrospray): [M+1] 240; [M+23] 262; [M-1] 238.

### EXAMPLE 5:

### (3S,5R)-3-Amino-5-methyl-octanoic acid hydrochloride(R)-2,6-Dimethyl-non-2-ene.

To (S)-citronellyl bromide (50 g, 0.228 mol) in THF (800 mL) at 0°C was added LiCl (4.3 g) followed by CuCl₂ (6.8 g). After 30 minutes methylmagnesium chloride (152 mL of a 3 M solution in THF, Aldrich) was added and the solution warmed to room temperature. After 10 hours the solution was cooled to 0°C and a saturated aqueous solution of ammonium chloride carefully added. The resultant two layers were separated and the aqueous phase extracted with ether. The combined organic phases were dried (MgSO₄) and concentrated to give (R)-2,6-dimethyl-non-2-ene. 32.6 g; 93%. Used without further purification. ¹H NMR (400 MHz; CDCl₃) δ 5.1 (m, 1H), 1.95 (m, 2H), 1.62 (s, 3H), 1.6 (s, 3H), 1.3 (m, 4H), 1.2 (m, 2H), 0.8 (s, 6H).

### (R)-4-Methyl-heptanoic acid.

To (R)-2,6-dimethyl-non-2-ene (20 g, 0.13 mol) in acetone (433 mL) was added a solution of CrO₃ (39 g, 0.39 mol) in H₂SO₄ (33 mL)/H₂O (146 mL) over 50 minutes. After 6 hours a further amount of CrO₃ (26 g, 0.26 mol) in H₂SO₄ (22 mL)/H₂O (100 mL) was added. After 12 hours the solution was diluted with brine and the solution extracted with ether. The combined organic phases were dried (MgSO₄) and concentrated. Flash chromatography (gradient of 6:1 to 2:1 hexane/EtOAc) gave (R)-4-methyl-heptanoic acid as an oil. 12.1 g; 65%. MS, *m*/*z* (relative intensity): 143 [M-H, 100%].

### (4R,5S)-4-Methyl-3-((R)-4-methyl-heptanoyl)-5-phenyl-oxazolidin-2-one.

To (R)-4-methyl-heptanoic acid (19 g, 0.132 mol) and triethylamine (49.9 g, 0.494 mol) in THF (500 mL) at 0°C was added trimethylacetylchloride (20 g, 0.17 mol). After 1 hour LiCl (7.1 g, 0.17 mol) was added followed by (4R,5S)-(+)-4-methyl-5-phenyl-2-oxazolidinone) 3 (30 g, 0.17 mol). The mixture was warmed to room temperature and after 16 hours the filtrate was removed by filtration and the solution concentrated under reduced pressure. Flash chromatography (7:1 hexane/EtOAc) gave (4R,5S)-4-methyl-3-((R)-4-methyl-heptanoyl)-5-phenyl-oxazolidin-2-one as an oil. 31.5 g; 79%. [α]_{D} = +5.5 (c 1 in CHCl₃).
MS, *m*/*z* (relative intensity): 304 [M+H, 100%].

### (3S,5R)-5-Methyl-3-((4R,5S)-4-methyl-2-oxo-5-phenyl-oxazolidine-3-carbonyl)-octanoic acid tert-butyl ester.

To (4R,5S)-4-methyl-3-((R)-4-methyl-heptanoyl)-5-phenyl-oxazolidin-2-one (12.1 g, 0.04 mol) in THF (200 ml) at -50°C was added sodium bis(trimethylsilyl)amide (48 mL of a 1 M solution in THF). After 30 min t-butylbromoaceate (15.6 g, 0.08 mol) was added. The solution was stirred for 4 hours at -50°C and then warmed to room temperature. After 16 hours a saturated aqueous solution of ammonium chloride was added and the two layers separated. The aqueous phase was extracted with ether and the combined organic phases dried (MgSO₄) and concentrated. Flash chromatography (9:1 hexane/EtOAc) gave (3S,5R)-5-methyl-3-((4R,5S)-4-methyl-2-oxo-5-phenyl-oxazolidine-3-carbonyl)-octanoic acid tert-butyl ester as a white solid 12 g; 72%. [α]_{D} = +30.2 (c 1 in CHCl₃). ¹³C NMR (100 MHz; CDCl₃) δ 176.47, 171.24, 152.72, 133.63, 128.87, 125.86, 80.85, 78.88, 55.34, 39.98, 38.77, 38.15, 37.58, 30.60, 28.23, 20.38, 20.13, 14.50, 14.28.

### (S)-2-((R)-2-Methyl-pentyl)-succinic acid 4-tert-butyl ester.

To (3S,5R)-5-methyl-3-((4R,5S)-4-methyl-2-oxo-5-phenyl-oxazolidine-3-carbonyl)-octanoic acid tert-butyl ester (10.8 g, 0.025 mol) in H₂O (73 mL) and THF (244 mL) at 0°C was added a premixed solution of LiOH (51.2 mL of a 0.8 M solution) and H₂O₂ (14.6 mL of a 30% solution). After 4 hours a further 12.8 mL LiOH (0.8 M solution) and 3.65 mL of H₂O₂ (30% solution) was added. After 30 minutes sodium bisulfite (7 g), sodium sulfite (13 g), and water (60 mL) was added followed by hexane (100 mL) and ether (100 mL). The two layers were separated and the aqueous layer extracted with ether. The combined organic phases were concentrated to an oil that was dissolved in heptane (300 mL). The resultant solid was filtered off and the filtrate dried (MgSO₄) and concentrated to afford (S)-2-((R))-2-methyl-pentyl)-succinic acid 4-tert-butyl ester (6 g, 93%) which was used immediately without further purification. MS, *m*/*z* (relative intensity): 257 [M+H, 100%].

### (3S, 5R)-3-Benzyoxycarbonylamlno-5-methyl-octanoic acid, tert-butyl ester.

A solution of (S)-2-((RF)-2-methyl-pentyl)-succinic acid 4-tert-butyl ester (6.0 g, 23.22 mmol) and triethylamine (3.64 mL, 26.19 mmol) in toluene (200 mL) was treated with diphenylphosphoryl azide (5.0 mL, 23.22 mL) and stirred at room temperature for 0.5 hours. After the reaction mixture was then heated at reflux for 3h and cooled briefly, benzyl alcohol was added (7.2 mL, 69.7 mmol) and the solution heated for another 3 h. After the reaction mixture was allowed to cool, it was diluted with ethyl ether (200 mL) and the combined organic layer was washed successively with saturated NaHCO₃ and brine and dried (Na₂SO₄). The concentrated organic component was purified by chromatography (MPLC) eluting with 8:1 hexanes: ethyl acetate to provide (3S, 5R)-3-benzyoxycarbonylamino-5-methyl-octanoic acid, *tert*-butyl ester (6.4 g, 75.8%). MS: M+1: 364.2, 308.2.

### (3S, 5R)-3-Amino-5-methyl-octanoic acid, tert-butyl ester.

A solution of (3S, 5R)-3-benzyoxycarbonylamino-5-methyl-octanoic acid, *tert*-butyl ester (2.14g, 5.88 mmol) in THF (50 mL) was treated with Pd/C (0.2 g) and H₂ at 50 psi for 2 hours. The reaction mixture was then filtered and concentrated to an oil in vacuo to give (3S, 5R)-3-amino-5-methyl-octanoic acid, *tert*-butyl ester in quantitative yield. MS: M+1: 230.2, 174.1.

### (3S, SR)-3-Amino-5-methyl-octanoic acid hydrochloride.

A slurry of (3S, 5R)-amino-5-methyl-octanoic acid, *tert*-butyl ester (2.59g, 11.3 mmol) in 6N HCl (100 mL) was heated under reflux 18 hours, cooled, and filtered over Celite. The filtrate was concentrated in vacuo to 25 mL and the resulting crystals were collected and dried to provide (3S, 5R)-3-amino-5-methyl-octanoic acid hydrochloride, mp 142.5-142.7°C (1.2g, 50.56%). A second crop (0.91g) was obtained from the filtrate. Anal. Calc'd for C₉H₁₉NO₂·HCl: C: 51.55, H: 9.61, N: 6.68, Cl: 16.91. Found: C: 51.69, H: 9.72, N: 6.56, Cl: 16.63.

### (3S, 5R)-3-Amino-5-methyl-octanoic acid hydrochloride acid salt.

5.3 g of 2S-(2R-methyl-pentyl)-succinic acid-4-tert-butyl ester contained in 30 mL methyltertbutyl ether is reacted at room temperature with 3.5 mL triethylamine followed by 6.4 g of diphenylphosphoryl azide. After allowing the reaction to exotherm to 45°C and stirring for at least 4 hours, the reaction mixture is allowed to cool to room temperature and stand while the phases separated. The lower layer is discarded and the upper layer is washed with water, followed by dilute aqueous HCl. The upper layer is then combined with 10 mL of 6 N aqueous HCl, and stirred at 45-65°C. The reaction mixture is concentrated by vacuum distillation to about 10 -14 mL and allowed to crystallize while cooling to about 5°C. After collecting the product by filtration, the product is washed with toluene and reslurried in toluene. The product is dried by heating under vacuum resulting in 2.9 g (67%) of white crystalline product. The product may be recrystallized from aqueous HCl. mp 137°C.

### EXAMPLE 6:

### Methanesulfonic acid (S)-3,7-dimethyl-oct-6-enyl ester.

To S-(-)-citronellol (42.8 g, 0.274 mol) and triethylamine (91 mL, 0.657 mol) in CH₂Cl₂ (800 mL) at 0°C was added methanesulphonyl chloride (26 mL, 0.329 mol) in CH₂Cl₂ (200 mL). After 2 hours at 0°C the solution was washed with 1 N HCl then brine. The organic phase was dried (MgSO₄) and concentrated to afford the titled compound an oil (60.5 g, 94%) which was used without further purification. MS, *m*/*z* (relative intensity): 139 [100%], 143 [100%].

### (R)-2,6-Dimethyl-oct-2-ene.

To methanesulfonic acid (S)-3,7-dimethyl-oct-6-enyl ester (60 g, 0.256 mol) in THF (1 L) at 0°C was added lithium aluminium hydride (3.8 g, 0.128 mol). After 7 hours, a further 3.8 g of lithium aluminum hydride was added and the solution warmed to room temperature. After 18 hours, a further 3.8 g of lithium aluminum hydride was added. After a further 21 hours, the reaction was carefully quenched with 1 N citric acid and the solution diluted further with brine. The resultant two phases were separated and the organic phase was dried (MgSO₄) and concentrated to afford the titled compound as an oil which was used without further purification. MS, *m*/*z* (relative intensity): 139 [M+H, 100%].

### (R)-4-Methyl-hexanoic acid.

A procedure similar to the synthesis of (R)-4-methyl-heptanoic acid was utilized giving the acid as an oil (9.3 g, 56%). MS, *m*/*z* (relative intensity): 129 [M-H, 100%].

### (4R,5S)-4-Methyl-3-((R)-4-methyl-hexanoyl)-5-phenyl-oxazolidin-2-one.

A procedure similar to the synthesis of (4R,5S)-4-methyl-3-((R)-4-methyl-heptanoyl)-5-phenyl-oxazolidin-2-one was utilized giving the titled compound as an oil (35.7 g, 95%). MS, *m*/*z* (relative intensity): 290 [M+H, 100.

### (35,5R)-5-Methyl-3-[1-((4R,5S)-4-methyl-2-oxo-5-phenyl-oxazolidin-3-yl)-methanoyl]-heptanoic acid tert-butyl ester.

A procedure similar to the preparation of (3S,5R)-5-methyl-3-((4R,5S)-4-methyl-2-oxo-5-phenyhxazolidine-3-carbonyl)-octanoic acid tert-butyl ester was followed giving the titled compound as an oil (7.48 g; 31%). MS, *m*/*z* (relative intensity): 178 [100%], 169 [100%]; [α]_{D} = + 21.6 (c 1 in CHCl₃).

### (S)-2-((R)-2-Methyl-butyl)-succinic acid 4-tert-butyl ester.

(3S,5R)-5-Methyl-3-[1-((4R,5S)-4-methyl-2-oxo-5-phenyl-oxazolidin-3-yl)-methanoyl]-heptanoic acid tert-butyl ester (7.26 g, 0.018 mol) in H₂O (53 mL) and THF (176 mL) at 0°C was added a premixed solution of LiOH (37 mL of a 0.8 M solution) and H₂O₂ (10.57 mL of a 30% solution) and the solution warmed to room temperature. After 2 hours sodium bisulfite (7 g), sodium sulfite (13 g), and water (60 mL) was added and the two layers were separated and the aqueous layer extracted with ether. The combined organic phases were concentrated to an oil that was dissolved in heptane (200 mL). The resultant solid was filtered off and the filtrate dried (MgSO₄) and concentrated to afford the titled compound as an oil (4.4 g) that was used without further purification. MS, *m*/*z* (relative intensity): 243 [100%].

### (3S, 5R)-3-Benzyoxycarbonylamino-5-methyl-heptanoic acid, tert-butyl ester.

This compound was prepared as described above starting with (S)-2-((R)-2-methyl-butyl) succinic acid, *4-tert*-butyl ester to give (3S, 5R)-3-benzyoxycarbonylamino-5-methyl-heptanoic acid, *tert*-butyl ester as an oil (73.3% yield). ¹H NMR (400 MHz; CDCl₃) δ 0.84(t, 3H, J = 7.33 Hz), 0.89(d, 3H, J = 6.60 Hz), 1.12-1.38 (m, 4H), 1.41 (s, 9H), 1.43-1.59 (m, 2H), 2.42 (m, 2H), 4.05 (m, 1H), 5.07 (t, 2H *J*=12.95 Hz), and 7.28-7.34 (m, 5H).

### (3S, 5R)-Amino-5-methyl-heptanoic acid, tert-butyl ester

This compound was prepared as described above starting with (3S, 5R)-3-benzyoxycarbonylamino-5-methyl-heptanoic acid, *tert*-butyl ester instead of (3S, 5R)-3-benzyoxycarbonylamino-5-methyl-octanoic acid, *tert*-butyl ester to give the titled compound. ¹H NMR (400 MHz; CDCl₃) δ 0.84 (overlapping t and d, 6H), 1.08-1.16(m, 2H), 1.27-1.30(m, 2H), 1.42(s, 9H), 1.62 (br s, 2H), 2.15 (dd, 1H, J= 8.54 and 15.62 Hz), 2.29(dd, 1H, J = 4.15 and 15.37 Hz), and 3.20(br s, 2H).

### (3S, 5R)-Amino-5-methyl-heptanoic acid hydrochloride

A slurry of (3S, 5R)-amino-5-methyl-heptanoic acid, *tert*-butyl ester (1.44g, 6.69 mmol) in 3N HCl was heated at reflux for 3 hours, filtered hot over Celite, and concentrated to dryness. Trituration of the resulting solid in ethyl ether provided (3S, 5R)-3-amino-5-methyl-heptanoic acid hydrochloride, (0.95g, 85%) mp 126.3-128.3°C.

### EXAMPLE 7:

### (3S, 5R)-3-Amino-5-methyl-nonanoic acid

(R)-4-Methyl-octanoic acid. Lithium chloride (0.39 g, 9.12 mmol) and copper (I) chloride (0.61 g, 4.56 mmol) were combined in 45 ml THF at ambient temperature and stirred 15 minutes, then cooled to 0°C at which time ethylmagnesium bromide (1 M solution in THF, 45 mL, 45 mmol) was added. (S)-citronellyl bromide (5.0 g, 22.8 mmol) was added dropwise and the solution was allowed to warm slowly to ambient temperature with stirring overnight. The reaction was quenched by cautious addition of sat. NH₄Cl (aq), and stirred with Et₂O and sat. NH₄Cl (aq) for 30 minutes. The phases were separated and the organic phase dried (MgSO₄) and concentrated. The crude (R)-2,6-dimethyl-dec-2-ene was used without purification. To a solution of (R)-2,6-dimethyl-dec-2-ene (3.8 g, 22.8 mmol) in 50 mL acetone at 0°C was added Jones' reagent (2.7 M in H₂SO₄ (aq), 40 mL, 108 mmol) and the solution was allowed to warm slowly to ambient temperature with stirring overnight. The mixture was partitioned between Et₂O and H₂O, the phases were separated, and the organic phase washed with brine, dried (MgSO₄), and concentrated. The residue was purified by flash chromatography (8:1 hexanes:EtOAc) to afford 2.14 g (59%) of the titled compound as a colourless oil: LRMS: *m*/*z* 156.9 (M+). Jones' reagent was prepared as a 2.7M solution by combining 26.7g CrO₃, 23 mL H₂SO₄, and diluting to 100 mL with H₂O.

### (4R, 5S)-4-Methyl-3-((R)-4-methyl-octanoyl)-5-phenyl-oxazolidin-2-one.

To (R)-4-methyl-octanoic acid (2.14 g, 13.5 mmol) in 25 mL CH₂Cl₂ at 0°C was added 3 drops DMF, followed by oxalyl chloride (1.42 mL, 16.2 mmol) resulting in vigorous gas evolution. The solution was warmed directly to ambient temperature, stirred 30 minutes, and concentrated. Meanwhile, to a solution of the oxazolidinone (2.64 g, 14.9 mmol) in 40 mL THF at -78°C was added n-butyllithium (1.6 M soln in hexanes, 9.3 mL, 14.9 mmol) dropwise. The mixture was stirred for 10 minutes at which time the acid chloride in 10 mL THF was added dropwise. The reaction was stirred 30 minutes at -78°C, then warmed directly to ambient temperature and quenched with sat. NH₄Cl. The mixture was partitioned between Et₂O and sat. NH₄Cl (aq), the phases were separated, and the organic phase dried (MgSO₄), and concentrated to furnish 3.2 g of the titled compound as a colourless oil. LRMS: *m*/*z* 318.2 (M+).

### (3S,5R)-5-Methyl-3-((4R,5S)-4-methyl-2-oxo-5-phenyl-oxazolidine-3-carbonyl)-nonanoic acid tert-butyl ester.

To a solution of diisopropylamine (1.8 mL, 12.6 mmol) in 30 mL THF at -78°C was added n-butyllithium (1.6 M soln in hexanes, 7.6 mL, 12.1 mmol), and the mixture stirred 10 minutes at which time (4R, 5S)-4-Methyl-3-((R)-4-methyl-octanoyl)-5-phenyl-oxazolidin-2-one (3.2 g, 10.1 mmol) in 10 mL THF was added dropwise. The solution was stirred for 30 minutes, *t*-butyl bromoacetate (1.8 mL, 12.1 mmol) was added quickly dropwise at -50°C, and the mixture was allowed to warm slowly to 10°C over 3 hours. The mixture was partitioned between Et₂O and sat. NH₄Cl (aq), the phases were separated, and the organic phase dried (MgSO₄), and concentrated. The residue was purified by flash chromatography (16:1 to 8:1 hexanes:EtOAc) to provide 2.65 g (61%) of the titled compound as a colourless crystalline solid, mp = 84-86°C. [δ]_{D}²³ +17.1 (c = 1.00, CHCl₃).

### (S)-2-((R)-2-Methyl-hexyl)-succinic acid 4-tert-butyl ester.

To a solution of (3S,5R)-5-Methyl-3-((4R,5S)-4-methyl-2-oxo-5-phenyl-oxazolidine-3-carbonyl)-nonanoic acid tert-butyl ester (2.65 g, 6.14 mmol) in 20 mL THF at 0°C was added a precooled (0°C) solution of LiOH monohydrate (1.0 g, 23.8 mmol) and hydrogen peroxide (30 wt% aqueous soln, 5.0 mL) in 10 mL H₂O. The mixture was stirred vigorously for 90 minutes, then warmed to ambient temperature and stirred 90 minutes. The reaction was quenched at 0°C by addition of 100 mL 10% NaHSO₃ (aq), then extracted with Et₂O. The phases were separated, and the organic phase washed with brine, dried (MgSO₄), and concentrated. The titled compound was used without purification.

### (3S, 5R)-3-Benzyoxycarbonylamino-5-methylnonanoic acid, tert-butyl ester

This compound, was prepared similarly as described above starting with (S)-2-((R)-2-methylhexyl) succinic acid, 4-*tert*-butyl ester instead of (S)-2-((R)-2-methylpentyl) succinic acid, 4-*tert*-butyl ester to provide the titled compound as an oil (71.6% yield). ¹HNMR (400 MHz; CDCl₃) δ 0.81(t, 3H, J = 4.40 Hz), 0.85(d, 3H, J = 6.55 Hz), 1.06-1.20(m, 7H), 1.36(s, 9H), 1.38-1.50(m, 2H), 2.36(m, 2H), 3.99(m, 1H), 5.02(m+s, 3H), and 7.28-7.28(m, 5H).

### (3S, 5R)-3-Amino-5-methyl-nonanoic acid, tert-butyl ester

This compound was prepared as described above starting with (3S, 5R)-benzyoxycarbonylamino-5-methyl-nonanoic acid, *tert*-butyl ester instead of (3S, 5R)-3-benzyoxycarbonylamino-5-methyl-octanoic acid, *tert*-butyl ester. Yield = 97%. ¹HNMR (400 MHz; CDCl₃) δ 0.82(overlapping d and t, 6H), 1.02-1.08(m, 1H), 1.09-1.36(m, 6H), 1.39(s, 9H), 1.47(br s, 1H), 1.80(s, 2H), 2.13(dd, 1H, J = 8.54 and 15.61 Hz), and 2.27(dd, 1H, J = 4.15 and 15.38 Hz).

### (3S, 5R)-3-Amino-5-methyl-nonanoic acid hydrochloride

A mixture of (3S, 5R)-3-amino-5-methyl-nonanoic acid, *tert*-butyl ester (1.50g, 6.16 mmol) in 3N HCl (100 mL) was heated at reflux for 3hours, filtered hot over Celite, and concentrated to 30mL in vacuo. The resulting crystals were collected, washed with additional 3N HCl, and dried to provide the title compound, mp 142.5-143.3°C. Additional crops were obtained from the filtrate to provide 1.03g (70.4%). Anal. Calc'd for C₁₀H₂₁NO₂-HCl: C: 53.68, H: 9.91, N: 6.26, Cl: 15.85. Found: C: 53.89, H: 10.11, N: 6.13. MS: M+1: 188.1.

### BIOLOGY EXAMPLES:

The biological activity of compounds suitable for use in the invention as alpha-2-delta ligands may be measured in a radioligand binding assay using [³H]gabapentin and the α-2-δ subunit derived from porcine brain tissues (Gee N.S., Brown J.P., Dissanayake V.U.K., Offord J., Thurlow R., Woodruff G.N., J. Biol. Chem., 1996;271:5879-5776).

### Example 1: Investigation into the effects of an alpha-2-delta ligand on the micturition reflex of anaesthetised rats.

### 1.0 Material and methods:

### Animals

Four caesarean derived (CD) rats weighing approximately 225 g were anaesthetised using urethane and continuous cystometry performed.

The animals were anaesthetised using urethane (1.2 g/kg, i.p.), Depth of anaesthesia was assessed by the stability of blood pressure and heart rate, and by an absence of hind limb withdrawal in response to paw pinch. Supplementary doses of urethane (0.1 g kg-1, i.v.) were given where necessary.

The trachea was intubated to maintain a patent airway, an external jugular vein was cannulated for drug administration, and a common carotid artery was cannulated with a heparinised cannula (20 units/ml heparin in 0.9% w/v saline) for the measurement of arterial blood pressure.

Blood pressure was measured using a pressure transducer and the heart rate (HR) derived electronically on-line from the blood pressure using PoneMah (Linton Pty Ltd UK). Body temperature was monitored with a rectal temperature probe and maintained between 36 - 38°C using a homeothermic blanket system (Harvard, UK). Animals were allowed to breathe air spontaneously throughout the duration of the experiment.

The urinary bladder was exposed by a midline abdominal incision. A cannula (c. 0.52 mm internal and 1.2 mm external diameter) was inserted into the bladder dome as a means of infusing the bladder while simultaneously recording intravesical bladder pressure. The bladder was infused with physiological saline (0.9%) at the rate of 0.046 ml/min, to simulate the maximal hourly diuresis rate of the rat (Klevmark B [1974] - Motility of the urinary bladder in cats during filling at physiological rates. I. Intravesical pressure patterns studied by a new method of cystometry. Acta Physlol Scand 90(3): 565-77). Following the surgical procedure the animals were allowed to stabilise for c. 30min. After the stabillsation period, cystometry was performed and bladder function (interval between micturition) in response to Gabapentin-HCl (0.3, 1, 3 and 10 mg/kg administered intravenously) was monitored.

### Statistical analysis

Differences between treatment groups were examined using ANOVA.

### Compound

Gabapentin-HCl (Tocris, UK) was dissolved in saline.

### 2.0 Results:

### Time interval between voiding episodes

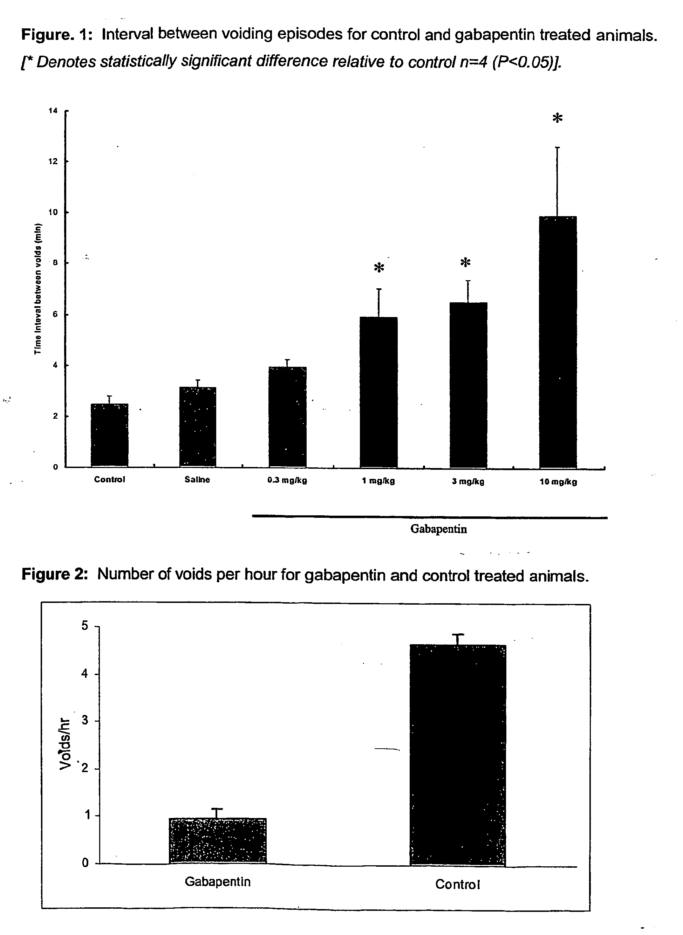

The interval between voiding events was increased in response to gabapentin (see Fig 1) relative to corresponding control periods. It was observed from the study that gabapentin significantly extended the interval between voiding events in a dose dependent manner. Indeed, 10 mg/kg of gabapentin administered intravenously significantly prolonged the micturition interval relative to corresponding control animals (3.1 ± 0.29 v 9.89 ± 2.72 min P<0.05). As a consequence of improving the interval between micturition events bladder capacity was improved significantly by 200% in response to gabapentin. It is interesting to note that gabapentin, when administered at 10 mg/kg, did not have any adverse effects on micturition efficiency, urine retention or bladder contraction upon voiding.

Thus it is demonstrated that administration of an alpha-2-delta ligand increases bladder capacity and reduces the LUTS in female animals, indicating the utility of such an approach in treating the LUTS associated with OAB.

### Example 2: Effect of an alpha-2-delta ligand on micturition frequency and bladder pressure in the male spontaneously hypertensive rat (SHR).

### 1.0 Materials and Methods:

Eight (8) male spontaneously hypertensive rats were prepared surgically with Data Sciences International (DSI) telemetry transponders (TL11M2-C50-PXT). Bladder pressure was continuously monitored with the aid of a pressure catheter placed via the dome of the bladder.

Bladder parameters (bladder pressure, voiding pressure and the number of voids) were recorded in response to placebo or treatment with 60 mg/kg Gabapentin administered subcutaneously (s/c).

### 2.0 Results:

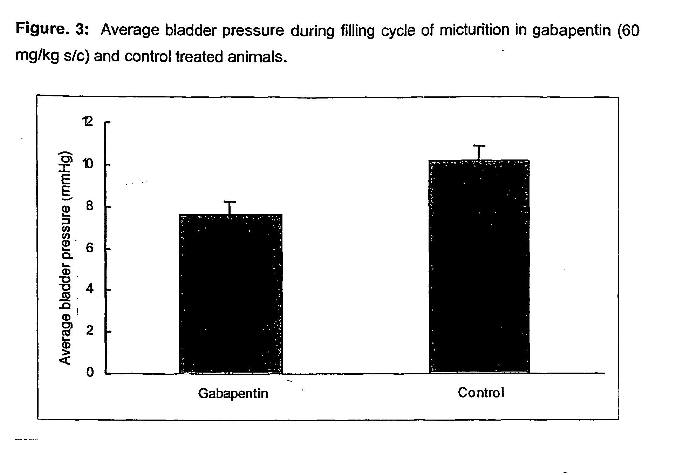

As depicted in Figure 2, Gabapentin at 60 mg/kg s/c significantly decreased (P<0.05) micturition interval and hence increased bladder capacity. Moreover, and importantly for lower urinary tract symptoms (LUTS), average bladder pressure during the filling stages of the micturition cycle were significantly decreased (P<0.05) in response to Gabapentin relative to corresponding control periods (see Figure 3).

Thus it is demonstrated that administration of an alpha-2-delta ligand increases bladder capacity and reduces the LUTS in male animals, indicating the utility of such an approach in treating the LUTS associated with BPH.

## Claims

1. Use of an alpha-2-delta ligand, or a pharmaceutically acceptable salt or solvate thereof, for the manufacture of a medicament for the treatment of LUTS, other than urinary incontinence, associated with OAB and/or BPH.

2. Use according to claim 1 wherein the LUTS is associated with BPH.

3. Use according to claim 1 wherein the LUTS is associated with OAB.

4. Use according to any one of claims 1 to 3, wherein the alpha-2-delta ligand is selected from: 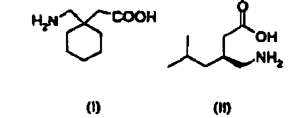 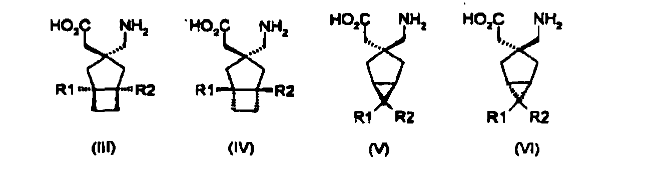 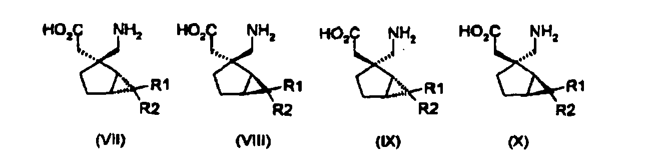 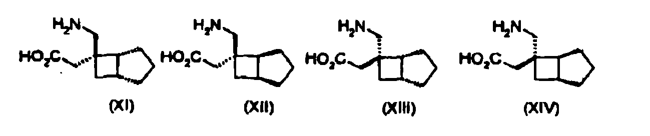 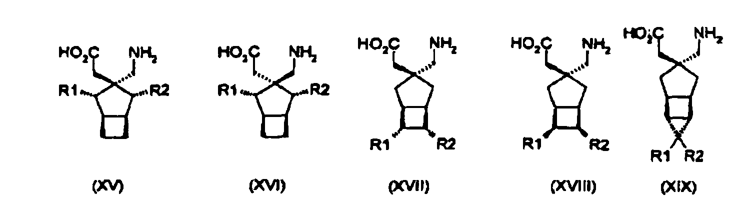 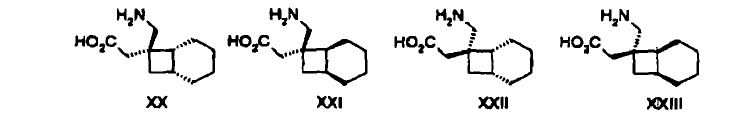 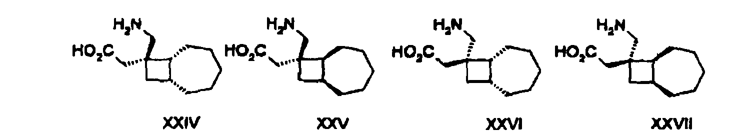 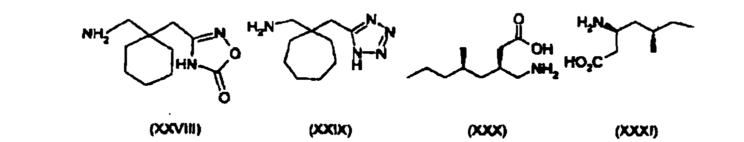 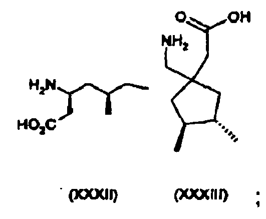 or a pharmaceutically acceptable salt or solvate thereof;
wherein R¹ and R² are each independently selected from H, straight or branched alkyl of 1-6 carbon atoms, cycloalkyl of from 3-6 carbon atoms, phenyl and benzyl, subject to the proviso that, except in the case of a tricyclooctane compound of formula (XVIII), R¹ and R² are not simultaneously hydrogen; or it is selected from 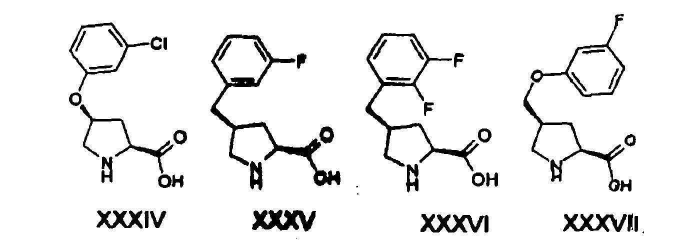 or a pharmaceutically acceptable salt or solvate thereof.

5. Use according to claim 4 wherein the alpha-2-delta ligand is gabapentin (I) 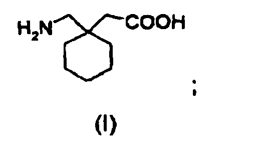 or a pharmaceutically acceptable salt or solvate thereof.

6. Use according to claim 4 wherein the alpha-2-delta ligand is pregabalin (II) 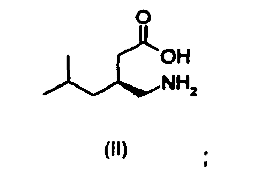 or a pharmaceutically acceptable salt or solvate thereof.

7. Use according to claim 4 wherein the alpha-2-delta ligand is (1α,3α,5α)(3-amino-methyl-bicyclo[3.2.0]hept-3-yl)-acetic acid (III') 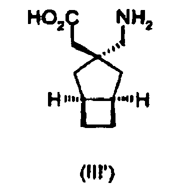 or a pharmaceutically acceptable salt or solvate thereof.

8. Use according to claim 4 wherein the alpha-2-delta ligand is 4-(3-fluorobenzyl)-pyrrolidine-2-carboxylic acid (XXXV) 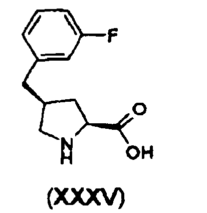 or a pharmaceutical acceptable salt or solvate thereof.

9. Use according to claim 4 wherein the alpha-2-delta ligand is a compound of the formula (XXX) as defined in claim 4, or a pharmaceutically acceptable salt or solvate thereof.

## Patentansprüche

1. Verwendung eines Alpha-2-Delta-Liganden oder eines pharmazeutisch verträglichen Salzes oder Solvats davon für die Herstellung eines Medikaments zur Behandlung von LUTS, anderer als Harninkontinenz, assoziiert mit OAB und/oder BPH.

2. Verwendung gemäß Anspruch 1, wobei LUTS mit BPH assoziiert ist.

3. Verwendung gemäß Anspruch 1, wobei LUTS mit OAB assoziiert ist.

4. Verwendung gemäß einem der Ansprüche 1 bis 2, wobei der Alpha-2-Delta-Ligand ausgewählt ist aus: 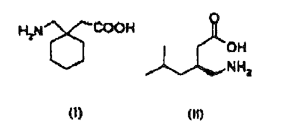 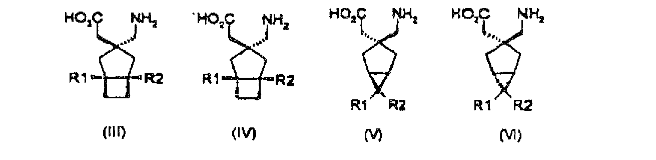 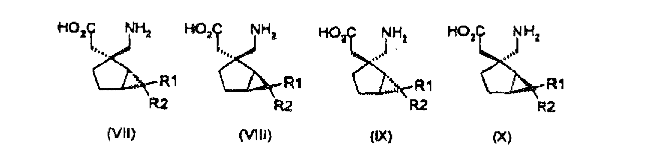 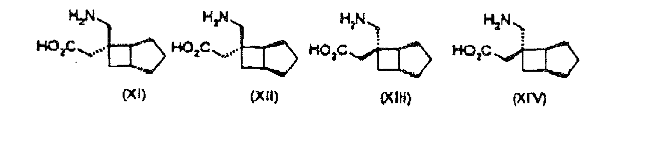 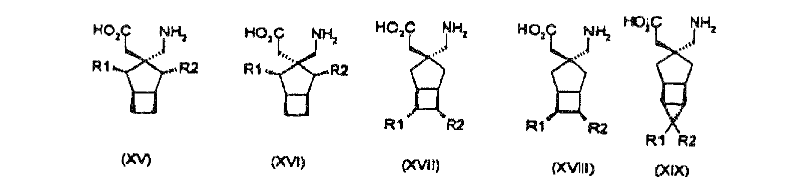 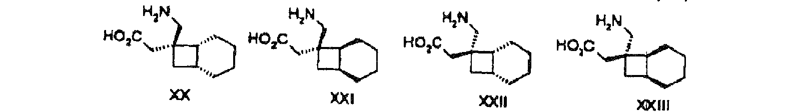 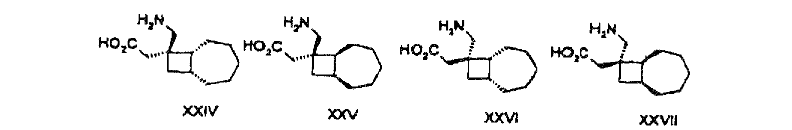 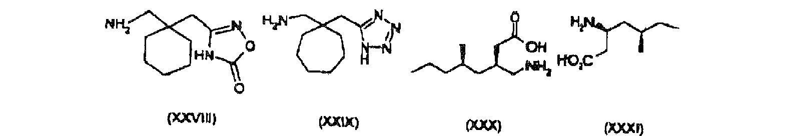 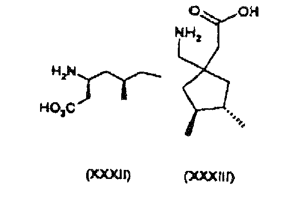 und einem pharmazeutisch verträglichen Salz oder Solvat davon;
wobei R¹ und R² jeweils unabhängig ausgewählt sind aus H, geradkettigem oder verzweigtem Alkyl mit 1 bis 6 Kohlenstoffatomen, Cycloalkyl mit 3 bis 6 Kohlenstoffatomen, Phenyl und Benzyl, mit der Maßgabe, dass, außer im Fall einer Tricyclooctan-Verbindung der Formel (XVIII) R¹ und R² nicht gleichzeitig Wasserstoff sind; oder er ausgewählt ist aus 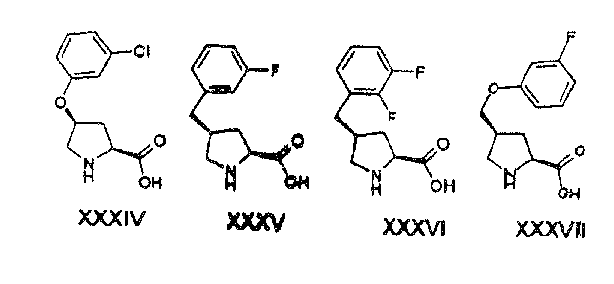 und einem pharmazeutisch verträglichen Salz oder Solvat davon.

5. Verwendung gemäß Anspruch 4, wobei der Alpha-2-Delta-Ligand Gabapentin (I) 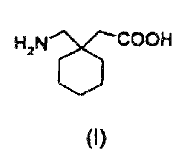 oder ein pharmazeutisch verträgliches Salz oder Solvat davon ist.

6. Verwendung gemäß Anspruch 4, wobei der Alpha-2-Delta-Ligand Pregabalin (II) 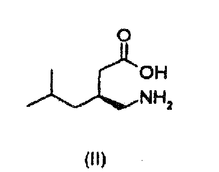 oder ein pharmazeutisch verträgliches Salz oder Solvat davon ist.

7. Verwendung gemäß Anspruch 4, wobei der Alpha-2-Delta-Ligand (1α,3α,5α)(3-Aminomethyl-bicyclo[3.2.0]hept-3-yl)essigsäure (III') 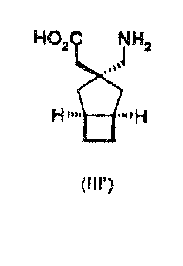 oder ein pharmazeutisch verträgliches Salz oder Solvat davon ist.

8. Verwendung gemäß Anspruch 4, wobei der Alpha-2-Delta-Ligand 4-(3-Fluorbenzyl)-pyrrolidin-2-carbonsäure (XXXV) 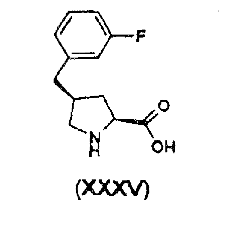 oder ein pharmazeutisch verträgliches Salz oder Solvat davon ist.

9. Verwendung gemäß Anspruch 4, wobei der Alpha-2-Delta-Ligand eine Verbindung der Formel (XXX), wie sie in Anspruch 4 definiert ist, oder ein pharmazeutisch verträgliches Salz oder Solvat davon ist.

## Revendications

1. Utilisation d'un ligand d'alpha-2-delta, ou d'un de ses sels ou produits de solvatation pharmaceutiquement acceptables, pour la production d'un médicament destiné au traitement du LUTS, autre que l'incontinence urinaire, associé à la OAB et/ou à la BPH.

2. Utilisation suivant la revendication 1, dans laquelle le LUTS est associé à la BPH.

3. Utilisation suivant la revendication 1, dans laquelle le LUTS est associé à la OAB.

4. Utilisation suivant l'une quelconque des revendications 1 à 3, dans laquelle le ligand d'alpha-2-delta est choisi entre : 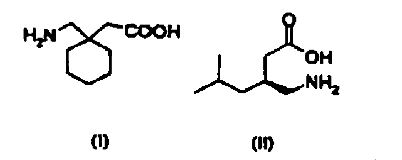 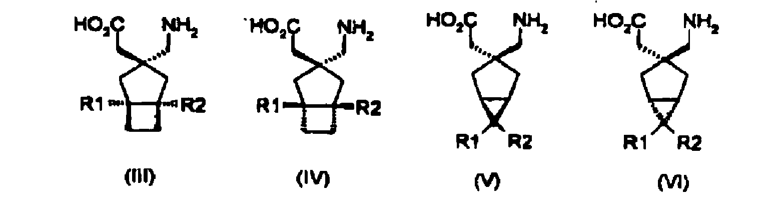 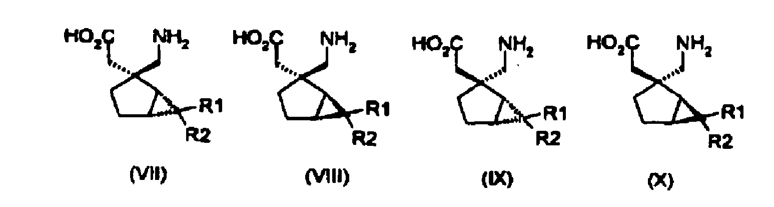 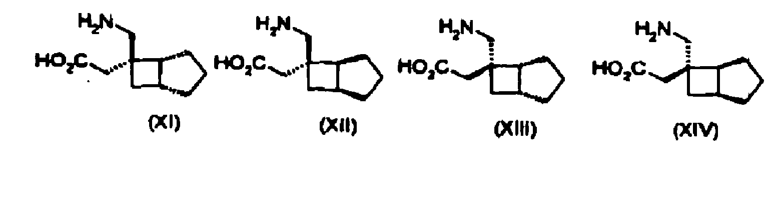 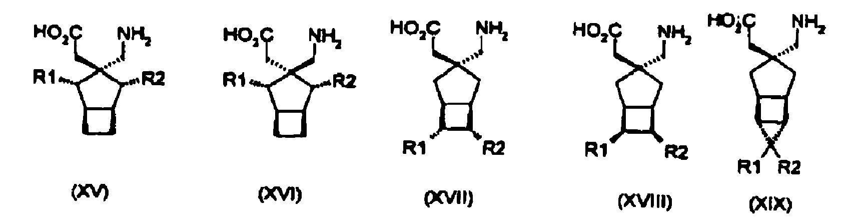 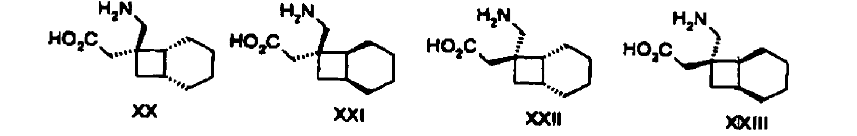 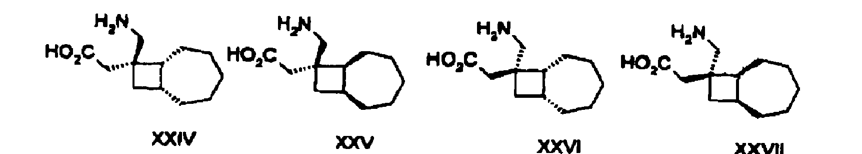 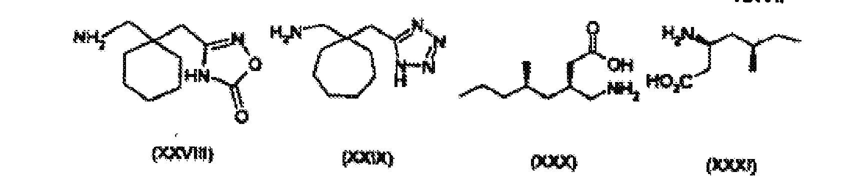 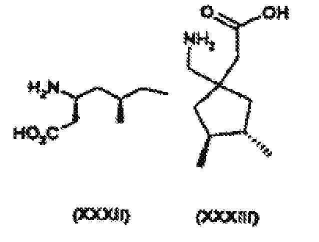 ou un de leurs sels ou produits de solvatation pharmaceutiquement acceptables ;
formules dans lesquelles R¹ et R² sont choisis chacun indépendamment entre H, des groupes alkyle droit ou ramifié ayant 1 à 6 atomes de carbone, cycloalkyle ayant 3 à 6 atomes de carbone, phényle et benzyle, sous réserve que, sauf dans le cas d'un dérivé de tricyclooctane de formule (XXVIII), R¹ et R² ne représentent pas simultanément un atome d'hydrogène ; ou bien est choisi entre 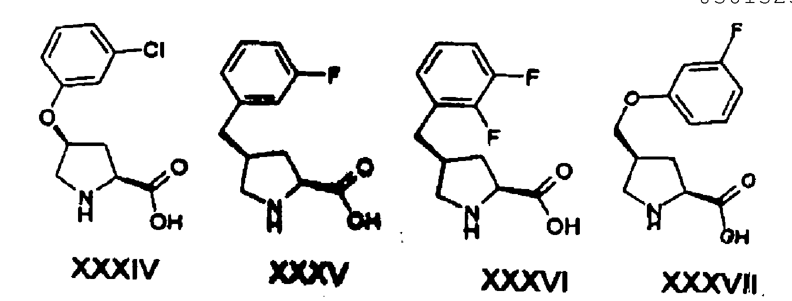 ou un de leurs sels ou produits de solvatation pharmaceutiquement acceptables.

5. Utilisation suivant la revendication 4, dans laquelle le ligand d'alpha-2-delta est la gabapentine (I) 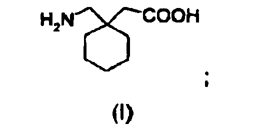 ou un de ses sels ou produits de solvatation pharmaceutiquement acceptables.

6. Utilisation suivant la revendication 4, dans laquelle le ligand d'alpha-2-delta est la prégabaline (II) 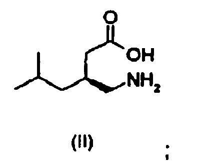 ou un de ses sels ou produits de solvatation pharmaceutiquement acceptables.

7. Utilisation suivant la revendication 4, dans laquelle le ligand d'alpha-2-delta est l'acide (1α,3α,5α)-(3-amino-méthyl-bicyclo[3.2.0]hept-3-yl)acétique (III') 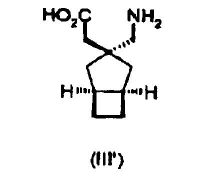 ou un de ses sels ou produits de solvatation pharmaceutiquement acceptables.

8. Utilisation suivant la revendication 4, dans laquelle le ligand d'alpha-2-delta est l'acide 4-(3-fluorobenzyl)-pyrrolidine-2-carboxylique (XXXV) 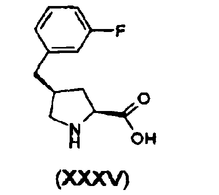 ou un de ses sels ou produits de solvatation pharmaceutiquement acceptables.

9. Utilisation suivant la revendication 4, dans laquelle le ligand d'alpha-2-delta est un composé de formule (XXX) répondant à la définition figurant dans la revendication 4, ou un de sels ou produits de solvatation pharmaceutiquement acceptables.
